# EUROPEAN PATENT SPECIFICATION

(11) **EP 2 109 605 B1**
(45) Date of publication and mention of the grant of the patent: **20.02.2013**
(21) Application number: 07866297.0
(22) Date of filing: 20.12.2007
(51) Int. Cl.: C07D 239/48, A61K 31/505, A61P 35/00

(54) **2- [(PHENYLAMINO) -PYRIMIDIN-4YLAMIN0] -CYCLOPENTANE CARBOXAMIDE DERIVATIVES AND RELATED COMPOUNDS AS INHIBITORS OF KINASES OF THE CELL CYCLE FOR THE TREATMENT OF CANCER**
2-[(PHENYLAMIN)-PYRIMIDIN-4YL-AMINO]-CYCLOPENTAN-CARBOXAMID-DERIVATE UND ENTSPRECHENDE VERBINDUNGEN ALS ZELLZYKLENKINASEHEMMER ZUR KREBSBEHANDLUNG
DERIVÉS 2-[(PHÉNYLAMINO)-PYRIMIDIN-4YL-AMIN0]-CYCLOPENTANE CARBOXAMIDE ET COMPOSÉS SIMILAIRES EN TANT QUE INHIBITEURS DES KINASES DU CYCLE CELLURAIRE POUR LE TRAITEMENT DU CANCER

(30) Priority: 22.12.2006 EP 06127043
(43) Date of publication of application: 21.10.2009
(73) Proprietor: Boehringer Ingelheim International GmbH, 55216 Ingelheim am Rhein (DE)
(72) Inventor: ZAHN, Stephan, 1130 Vienna (AT); BOEHMELT, Guido, 2531 Gaaden (AT); GUERTLER, Ulrich, 1120 Vienna (AT); MANTOULIDIS, Andreas, 1120 Vienna (AT); SCHOOP, Andreas, 82061 Neuried (DE); SOLCA, Flavio, 1230 Vienna (AT); TONTSCH-GRUNT, Ulrike, 2500 Baden (AT); TREU, Matthias, 1110 Vienna (AT)
(74) Representative: Simon, Elke Anna Maria
(86) International application number: PCT/EP2007/064342
(87) International publication number: WO 2008/077885

(56) References cited:
- WO-A-2005/063722
- WO-A-2005/118544
- WO-A-2007/003596
- WO-A-2007/027238

## Description

The present invention relates to new compounds, the isomers thereof, processes for preparing these pyrimidines and their use as medicaments.

Pyrimidines are generally known as inhibitors ofkinases. Thus, for example, substituted pyrimidines with a non-aromatic group in the 4-position are described as active components with an anticancer activity in International Patent Applications WO 02/096888 and WO 03/032997. Further pyrimidines are disclosed in WO 2005/063722, WO 2005/118544, WO 2007/003596 and WO 2007/027238.

The aim of the present invention is to indicate new active substances that can be used for the prevention and/or treatment of ailments characterised by excessive or abnormal cell proliferation.

### Detailed description of the invention

It has now surprisingly been found that example compounds 1-41 act as inhibitors of specific cell cycle kinases. Thus the compounds according to the invention may for example be used for the treatment of diseases connected with the activity of specific cell cycle kinases and characterised by excessive or abnormal cell proliferation.

In another aspect the invention relates to example compounds 1-41, or the pharmaceutically effective salts thereof, for use as pharmaceutical compositions.

In another aspect the invention relates to example compounds 1-41, or the pharmaceutically effective salts thereof, for preparing a pharmaceutical composition with an antiproliferative activity.

In another aspect the invention relates to a pharmaceutical preparations, containing as active substance one or more compounds of example compounds 1-41, or the pharmaceutically effective salts thereof, optionally in combination with conventional excipients and/or carriers.

In another aspect the invention relates to the use of example compounds 1-41 for preparing a pharmaceutical composition for the treatment and/or prevention of cancer, infections, inflammations and autoimmune diseases.

In another aspect the invention relates to a pharmaceutical preparation comprising an example compound 1-41, optionally in the form of the tautomers, the racemates, the enantiomers, the diastereomers and the mixtures thereof, and optionally the pharmaceutically effective salts thereof and at least one other cytostatic or cytotoxic active substance different from compounds 1-41.

### List of abbreviations

| | | | |
|---|---|---|---|
| eq | equivalent(s) | cat., cat | catalyst, catalytic |
| Ac | acetyl | conc. | concentrated |
| Boc | t-butyloxycarbonyl | LC | liquid chromatography |
| resp. | respectively | min | minute(s) |
| cHex | cyclohexane | Me | methyl |
| TLC | thin layer chromatography | MeCN | acetonitrile |
| DCM | dichloromethane | MS | mass spectrometry |
| DMF | *N,N*-dimethylformamide | NMP | *N*-methylpyrrolidone |
| DMA | *N,N*-dimethylacetamide | NMR | nuclear magnetic resonance |
| DMSO | dimethylsulphoxide | R_{f} (Rf) | retention factor |
| EE | ethyl acetate | RP | reversed phase |
| ESI | electron spray ionization | RT | ambient temperature or retention time (HPLC) |
| EtOH | ethanol | TEMPO | 2,2,6,6-tetramethyl-1-piperidin-*N*-oxyl radical |
| MeOH | methanol | *tert* | tertiary |
| h | hour(s) | THF | tetrahydrofuran |
| HATU | 2-(1H-7-azabenzotriazol-1-yl)-1,1,3,3-tetramethyluronium hexafluorophosphate | TBTU | *O*-(benzotriazol-1-yl)-*N,N,N',N'*-tetramethyl-uronium tetrafluoroborate |
| HPLC | high performance liquid chromatography | UV | ultraviolet |
| Hünig base | *N*-ethyl-diisopropylamine | aqu. | aqueous |

### General

Unless stated to the contrary, all the reactions are carried out in commercially obtainable apparatus using methods conventional in chemical laboratories. The solvents used are purchased in *pro analysi* quality and used without further purification. All the reagents are used directly in the synthesis without further purification.

Air- and/or moisture-sensitive starting materials are stored under argon and corresponding reactions and manipulations with them are carried out under protective gas (nitrogen or argon).

### Chromatography

For the preparative medium pressure chromatography (MPLC, normal phase) silica gel obtained from Millipore (Granula Silica Si-60A 35-70 µm) or C-18 RP silica gel (RP-phase) obtained from Macherey Nagel (Polygoprep 100-50 C18) is used.

The thin layer chromatography is carried out on ready-made silica gel 60 TLC plates (with fluorescence indicator F-254) made by Merck.

For the preparative HPLC, columns made by Waters (XTerra Prep. MS C 18, 5 µM, 30*100 mm or XTerra Prep. MS C18, 5 µm, 50* 100 mm OBD or Symmetrie C18, 5 µm, 19*100 mm), are used, for example, the analytical HPLC (reaction monitoring) is carried out with columns made by Agilent (Zorbax SB-C8, 5 µm, 21.2*50mm).

### HPLC-mass spectroscopy/UV-spectrometry

The retention times/MS-ESI⁺ for characterising the Examples are produced using an HPLC-MS apparatus (high performance liquid chromatography with mass detector) made by Agilent, which is operated by an "acidic" or "basic" method as described below:

### Method 1 (acidic):

The apparatus is constructed so that the chromatography (column: XTerra MS C18, 2.5 µm, 2.1*30 mm, Messrs. Waters, Part.No.186000592) is followed by a diode array detector (G1315B made by Agilent) and a mass detector (1100 LS-MSD SL; G1946D; Agilent) connected in series.

This apparatus is operated with a flow of 1.1 mL/min. For a separation process a gradient is run through within 3.1 min (gradient at the start: water/MeCN 95/5, gradient at the finish: water/MeCN 5/95; HCOOH (formic acid) is added to the water to produce a 0.1 % solution (v/v).

### Method 2 (basic):

The apparatus is constructed so that the chromatography (column: Gemini MS C18, 3 µm, 2*20 mm, made by Phenomenex, Part No.: 00M-4439-B0-CE) is followed by a diode array detector (G1315B made by Agilent) and a mass detector (1100 LS-MSD SL; G1946D; Agilent) connected in series.

This apparatus is operated with a flow of 1.1 mL/min. For a separation process a gradient is run through within 3.1 min (gradient at the start: Solvent A/MeCN 95/5, gradient at the finish: Solvent A/MeCN 5/95; Solvent A consists of an aqu. Solution of the following composition: 5 mM NH₄HCO₃/20 mM NH₃, with a pH of 9.3.

Where the preparation of the starting compounds has not been described, these are commercially obtainable or may be produced analogously to known compounds or methods described herein. Substances described in the literature are prepared using the methods of synthesis published.

### Preparation of the compounds according to the invention

The compounds according to the invention may be prepared using the method of synthesis described hereinafter, the substituents of the general formulae having the meanings mentioned hereinbefore.

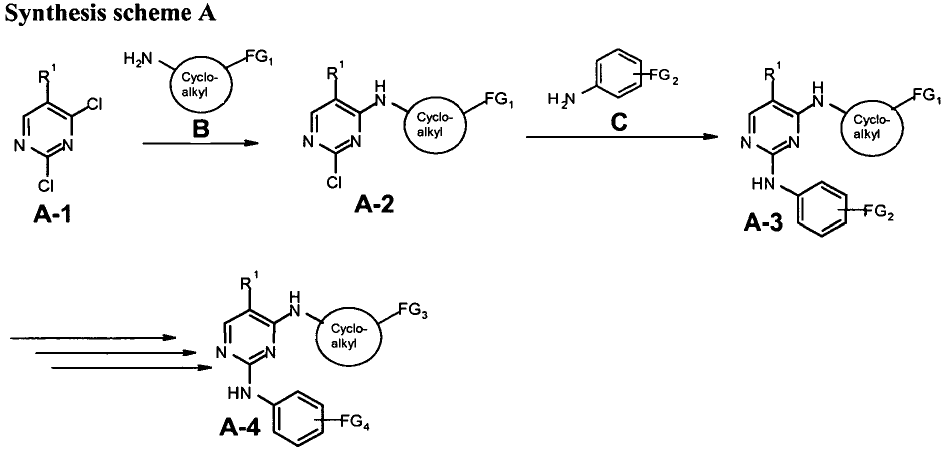

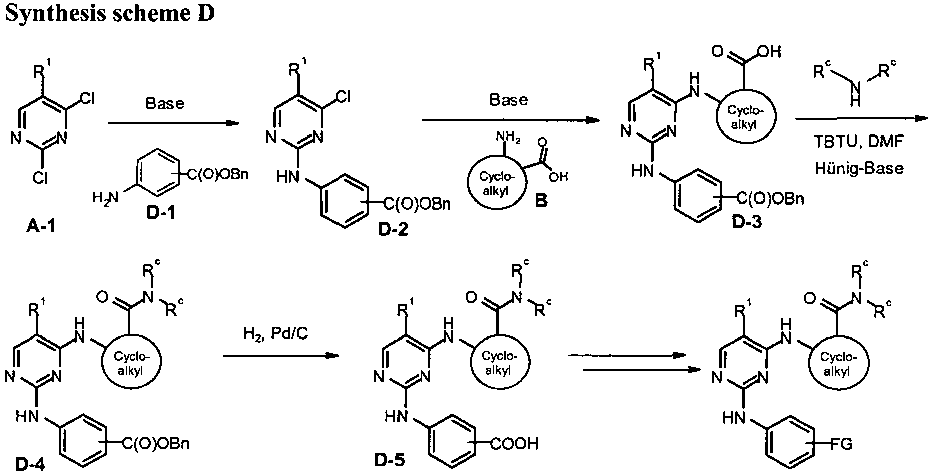

Optionally in the course of the synthesis schemes it is also possible to carry out one or more transformations of one or more functional groups (FG). This is described in the Examples, where relevant.

### Preparation of the synthesis components A - D

### A-1) 2,4-Dichloro-5-trifluoromethyl-pyrimidine

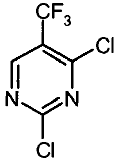

48 g (267 mmol) 5-trifluoromethyluracil are suspended in 210 mL phosphorus oxychloride (POCl₃) while moisture is excluded. 47.7 g (320 mmol, 1.2 eq) diethylaniline are added dropwise to this suspension so slowly that the temperature remains between 25°C and 30°C. After the addition has ended the mixture is stirred for another 5 - 10 min in the water bath and heated for 5 - 6 h at 80 - 90°C while moisture is excluded. The excess POCl₃ is destroyed by stirring into approx. 1200 g sulphuric acid-containing ice water and the aqueous phase is immediately extracted 3 times with in each case 500 mL ether or *tert-*butyl-methyl-ether. The combined ethereal extracts are washed twice with 300 mL sulphuric acid-containing ice water (approx. 0.1 M) and with cold saline solution and immediately dried on sodium sulphate. The desiccant is filtered off and the solvent is eliminated in vacuo. The residue is distilled *in vacuo* (10 mbar) through a short column (20 cm) (head temperature: 65 - 70°C), to obtain 35.3 g of a liquid which is poured off under protective gas and stored.
TLC: R_{f} = 0.83 (cHex:EE = 3:1)

5-Halo-2,4-dichloropyrimidines may be prepared analogously from the uracil derivatives or are commercially obtainable.

### A-2a) (1S,2R)-2-(2-Chloro-5-trifluoromethyl-pyrimidin-4-ylamino)cyclopentanecarboxylic acid isopropylamide

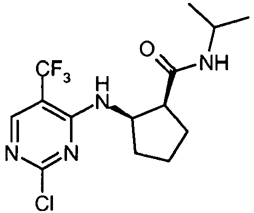

10.6 g (48.9 mmol) **A-1** are dissolved in 75 mL DMA and then 7.5 g (44 mmol, 0.9 eq) (1S, 2R)-2-amino-cyclopentanecarboxylic acid-isopropylamide **(B-2)** are added. After the addition of 69.5 mL (406.2 mmol, 8.3 eq) N-ethyl-diisopropylamine the reaction mixture is stirred for 1 h at 70°C. After this time, total conversion can be demonstrated by thin layer chromatography. After cooling to RT the reaction mixture is combined with silica gel and the volatile constituents are eliminated in vacuo. 4.2 g (11.9 mmol, 24%) **A-2a** are obtained by purification by column chromatography (normal phase, silica gel, cHex/EE 85/15).
R_{f} = 0.17 (silica gel, cHex:EE 3:1)
RT = 2.01 min
MS-ESI⁺: 351 (M+H)⁺

### A-2b) (1S,2R)-2-(2-Chloro-5-fluoro-pyrimidin-4-ylamino)cyclopentanecarboxylic acid-isopropylamide

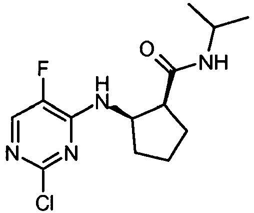

5.7 g (34.3 mmol, 1.4 eq) 5-fluoro-2,4-dichloropyrimidine are dissolved in 650 mL isopropanol and first of all 47.8 g (345.6 mmol, 14.3 eq) potassium carbonate and then 4 g (24.2 mmol, 1 eq) (1S, 2R)-2-aminocyclopentanecarboxylic acid are added with vigorous stirring. The reaction mixture is stirred for 5 h at 50°C until total conversion is obtained. The reaction mixture is concentrated to dryness in vacuo, the residue is taken up in 400 mL water and washed 3 times with 50 mL DCM. The organic phase is discarded and the aqueous phase is adjusted to pH 2 with conc. aqueous hydrochloric acid while stirring vigorously. The precipitate formed is filtered off and dried in vacuo. The mother liquor is extracted 3 times with EE, the combined organic phases are dried on magnesium sulphate and the volatile constituents are eliminated in vacuo. A total of 5.5 g (21.0 mmol, 87%) (1S,2R)-2-(2-chloro-5-fluoro-pyrimidin-4-ylamino)cyclopentanecarboxylic acid are obtained.

3.3 g (12.6 mmol, 1 eq) (1S,2R)-2-(2-chloro-5-fluoro-pyrimidin-4-ylamino) cyclopentanecarboxylic acid are suspended in 300 mL DCM, 10.8 g (62.8 mmol, 5 eq) Hünig base are added to the suspension and the solution formed is combined successively with 5.6 g (17.6 mmol, 1.4 eq) TBTU and 1.4 mL (16.6 mmol, 1.3 eq) isopropylamine. The reaction mixture is stirred for 2 h at RT, and after this time total conversion can be observed by monitoring the reaction. The reaction mixture is freed from all volatile constituents *in vacuo* using the rotary evaporator, the residue is taken up in 10 mL of DMF and the solution is poured into 500 mL water. It is made basic with potassium carbonate, the precipitate formed is filtered off, dissolved in DCM and dried on magnesium sulphate. After all the volatile constituents have been eliminated in vacuo, 3.4 g (11.4 mmol, 90%) **A-2b** is obtained.
RT = 1.67 min
MS-ESI⁺: 301 (M+H)⁺

### A-2c) (1S,2R)-2-(2,5-Dichloropyrimidin-4-ylamino)cyclopentanecarboxylic acid isopropylamide

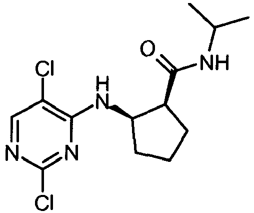

3.9 g (34.3 mmol, 1.4 eq) 2,4,5-trichloropyrimidine are dissolved in 650 mL isopropanol and first of all 47.8 g (345.6 mmol, 14.3 eq) potassium carbonate and then 4 g (24.2 mmol, 1 eq) (1S, 2R)-2-aminocylclopentanecarboxylic acid are added with vigorous stirring. The reaction mixture is stirred for 4.5 h at 50°C, then monitoring of the reaction by thin layer chromatography indicates total conversion. The reaction mixture is concentrated to dryness in vacuo, the residue is taken up in 400 mL water and washed 3 times with 50 mL DCM. The organic phase is discarded, the aqueous phase is adjusted to pH 2 with conc. aqueous hydrochloric acid while stirring vigorously. The precipitate formed is filtered off and dried in vacuo. 5.2 g (18.7 mmol, 77%) (1S,2R)-2-(2,5-dichloropyrimidin-4-ylamino)cyclopentanecarboxylic acid are obtained.
RT = 1.74 min
MS-ESI⁺: 276 (M+H)⁺

5.2 g (18.7 mmol, 1 eq) (1S,2R)-2-(2,5-dichloropyrimidin-4-ylamino) cyclopentanecarboxylic acid are suspended in 300 mL DCM, 16.1 mL (93.6 mmol, 5 eq) Hünig base are added to the suspension and the resulting solution is combined successively with 8.4 g (26.2 mmol, 1.4 eq) TBTU and 2.1 mL (24.7 mmol, 1.3 eq) isopropylamine. The reaction mixture is stirred for approx. 2 h at RT until total conversion is obtained. The reaction mixture is freed from all volatile constituents using the rotary evaporator, the residue is taken up in a little DMF and the solution is poured into 1 L water, then made basic with potassium carbonate, the precipitate formed is filtered off and dried in vacuo. 5.3 g (16.6 mmol, 89%) **A-2c** are obtained.
RT = 1.87 min
MS-ESI⁺: 317 (M+H)⁺

### A-2d) (1S,2R)-2-(5-Bromo-2-chloropyrimidin-4-ylamino)cyclopentanecarboxylic acid isopropylamide

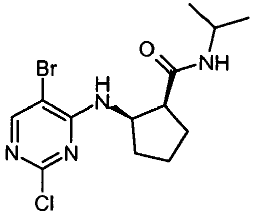

2.2 g (9.7 mmol, 1 eq) 5-bromo-2,4-dichloropyrimidine are dissolved in 14 mL 1-butanol, the solution is combined with 2 g (11.7 mmol, 1.2 eq) (1S, 2R)-2-aminocyclopentane-carboxylic acid-isopropylamide and after the addition of 8.3 mL (48.3 mmol, 5 eq) Hünig base the reaction mixture is stirred for 2 h at 70°C. The reaction mixture is freed from all volatile constituents after cooling in vacuo and the solid obtained is digested in a little MeOH. The solid is suction filtered and the mother liquor is again evaporated down in vacuo and poured onto 100 mL water. The solid obtained is suction filtered and combined with the product obtained first. In all: 3.1 g (8.5 mmol, 89%) **A-2d** are obtained.
RT = 1.88 min
MS-ESI⁺: 361/363 (M+H)⁺

### A-2e) (1S,2R)-2-(5-Bromo-2-chloropyrimidin-4-ylamino)cyclopentanecarboxylic acid-methylamide

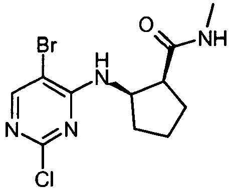

15.5 g (68.2 mmol, 1 eq) 5-bromo-2,4-dichloropyrimidine are dissolved in 900 mL isopropanol and first of all 88 g (680.9 mmol, 10 eq) potassium carbonate and then 11.5 g (68.2 mmol, 1 eq) (1S, 2R)-2-aminocylclopentanecarboxylic acid are added with vigorous stirring. The reaction mixture is stirred for 16 h at 50°C, then monitoring of the reaction by TLC indicates total conversion. The reaction mixture is evaporated to dryness in vacuo, the residue is taken up in 1 L water and washed 3 times with 350 mL of DCM. The organic phase is discarded, the aqueous phase is adjusted to pH 2 with conc. aqueous hydrochloric acid while stirring vigorously. The precipitate formed is filtered off, taken up in EE and the organic phase is dried on magnesium sulphate. After all the volatile constituents have been eliminated *in vacuo,* 15.6 g (48.7 mmol, 71%) (1S,2R)-2-(2-chloro-5-bromopyrimidin-4-ylamino)cyclopentanecarboxylic acid is obtained.
RT = 1.78 min
MS-ESI⁺: 320/322 (M+H)⁺

5 g (15.6 mmol, 1 eq) (1S,2R)-2-(2-chloro-5-bromopyrimidin-4-ylamino) cyclopentanecarboxylic acid are suspended in 200 mL DCM, 15.2 mL (109.2 mmol, 7 eq) triethylamine are added to the suspension and the resulting solution is combined successively with 7.1 g (18.7 mmol, 1.2 eq) HATU and 3.2 g (46.8 mmol, 3 eq) methylamine-hydrochloride. The reaction mixture is stirred for 16 h at RT, and after this time virtually total conversion can be observed by monitoring of the reaction. The reaction mixture is washed successively with two lots of 200 mL of 1N NaOH (aq) and 200 mL water and the organic phase is dried on magnesium sulphate. After the elimination of all the volatile constituents in vacuo, 5.4 g (14.6 mmol, content 90%, 93%) **A-2e** are obtained.
RT = 1.69 min
MS-ESI⁺: 333/335 (M+H)⁺

### B-1) (1S,2R)-2-Aminocyclopentanecarboxylic acid hydrochloride

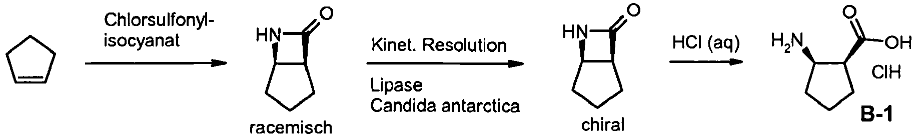

The synthesis is carried out according to the reaction plan described in the literature (Forro, E. And Fueloep, F. (2003) Lipase-Catalyzed Enantioselective Ring Opening of Unactivated Alicyclic-Fused β-Lactams in an Organic Solvent. Org. Lett. 5, 1209-1212). Racemic cis-2-aminocyclopentanecarboxylic acid may also be obtained by acidic, hydrolytic ring opening of the racemic lactam.

### B-2) (1S,2R)-2-Aminocyclopentanecarboxylic acid isopropylamide

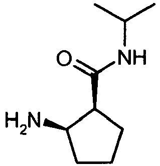

1.2 g (7.2 mmol, 1 eq) (1S, 2R)-2-aminocyclopentanecarboxylic acid hydrochloride **B-1** are combined with 15.4 mL (181.2 mmol, 25 eq) isopropylamine and then 3.6 g (10.9 mmol, 1.5 eq) TBTU are added with stirring. After approx. 30 min the mixture is combined with 9 mL of DMF and the reaction mixture is stirred for 16 h at RT. TLC indicates total conversion. The reaction mixture is filtered through basic aluminium oxide, washed with DMF and MeOH and the filtrate is combined with silica gel. After elimination of all the volatile constituents in vacuo, 1.2 g (7.2 mmol, 99 %) **B-2** are obtained by purification by column chromatography (normal phase, silica gel, DCM/MeOH/NH₃ 95/5/0.5).
R_{f} = 0.41 (silica gel, DCM/MeOH/NH₃ 3/1/0.1)
MS-ESI⁺: 171 (M+H)⁺

### C-1) 4-Amino-dimethylbenzylamine

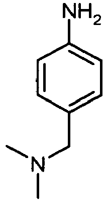

7 g (46.3 mmol, 1 eq) 4-nitrobenzaldehyde are dissolved in 1,2-dichloroethane, then 27.7 mL of dimethylamine (55.6 mmol, 1.2 eq, 2 molar solution in THF) are added and the solution is stirred for 10 min at RT. To this are then added, successively, 3.2 mL (55.6 mmol, 1.2 eq) acetic acid and 14.7 g (69.5 mmol, 1.5 eq) sodium trisacetoxyborohydride and the reaction mixture is stirred for 1 h at RT. The reaction mixture is slowly poured into saturated, aqueous sodium hydrogen carbonate solution. The aqueous phase is washed twice with 100 mL of DCM and the combined organic phases are dried on magnesium sulphate. 6.9 g (38.1 mmol, 82%) 4-nitro-dimethylbenzylamine are obtained, which is used in the following reduction without any further purification.
MS-ESI⁺: 181 (M+H)⁺

6.9 g (38.1 mmol) 4-nitro-dimethylbenzylamine are dissolved in 100 mL MeOH, combined with 1 g of palladium on activated charcoal (10 % Pd) and stirred for 16 h under a hydrogen atmosphere (5 bar) in the autoclave. After this time, total conversion can be detected (monitoring of reaction by HPLC). The catalyst is filtered off and all the volatile constituents are eliminated in vacuo. 6.8 g crude **C-1** are obtained, which is used without further purification in the syntheses of Examples **1, 2** and **6.**
MS-ESI⁺: 151 (M+H)⁺

### C-2) tert-Butyl 4-[1-(4-aminophenyl)-piperidin-4-yl]-piperazine-1-carboxylate

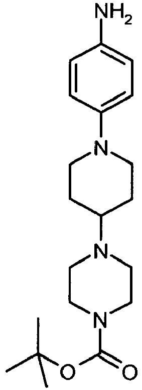

38.2 g (205.1 mmol, 1 eq) 1-Boc-piperazine are dissolved in 350 mL THF and 37 mL (207.0 mmol, 1 eq) 1-benzyl-4-piperidone are added. The mixture is acidified to a pH of 5 with acetic acid and while cooling with ice 23.2 g (109.5 mmol, 0.53 eq) sodium trisacetoxyborohydride are slowly added. The mixture is stirred for 16 h at RT and the reaction mixture is then adjusted with aqueous potassium carbonate solution to pH 9-10. The organic phase is separated off, the aqueous phase is extracted 3 times with 150 mL EE and after the organic phases have been combined they are dried on sodium sulphate. After all the volatile constituents have been eliminated in vacuo 64.3 g (178.8 mmol, 87%) *tert-*butyl 4-(1-benzyl-piperidin-4-yl)-piperazine-1-carboxylate is obtained.
MS-ESI⁺: 360 (M+H)⁺
64.3 g (178.8 mmol) tert. Butyl 4-(1-benzyl-piperidin-4-yl)-piperazine-1-carboxylate are dissolved in 500 mL MeOH, combined with 3.2 g palladium on activated charcoal (3.01 mmol, 0.02 eq, 10% Pd) and the reaction mixture is stirred for a total of 7 days under a hydrogen atmosphere (8 bar) in an autoclave at RT. After the catalyst has been filtered off and all the volatile constituents have been eliminated in vacuo, 39.1 g (145.3 mmol, 81%) tert. Butyl 4-piperidin-4-yl-piperazine-1-carboxylate are obtained.
MS-ESI⁺: 270 (M+H)⁺
10 g (70.9 mmol, 1 eq) of4-fluoronitrobenzene are dissolved in 20 mL isopropanol, combined with 19.1 g (70.9 mmol, 1 eq) *tert.* butyl 4-piperidin-4-yl-piperazine-1-carboxylate and after the addition of 12.5 ml (89.8 mL, 1.3 eq) triethylamine the reaction mixture is heated to 160°C for 10 min with stirring (microwave). The reaction mixture is evaporated down in vacuo, the residue is taken up in MeOH/DCM, combined with silica gel and 20.5 g (52.4 mmol, 74 %) tert. Butyl 4-[1-(4-nitro-phenyl)-piperidin-4-yl]-piperazine-1-carboxylate are obtained by purification by column chromatography (normal phase, silica gel, DCM/MeOH/NH₃ 95/5/0.5).
4.16 g (10.65 mmol) tert. Butyl 4-[1-(4-nitro-phenyl)-piperidin-4-yl]-piperazine-1-carboxylate are dissolved in a mixture of 100 mL MeOH and 75 mL DCM, combined with Raney nickel and stirred in the autoclave under a hydrogen atmosphere (10 bar) for 36 h at RT until a total conversion is observed. The catalyst is filtered off, and all the volatile constituents are eliminated in vacuo. 3.78 g (10.5 mmol, 99 %) **C-2** are obtained, which is used without further purification.
MS-ESI⁺: 361 (M+H)⁺

### C-3) 1-(4-Aminophenyl)-3-methyl-imidazolidin-2-one

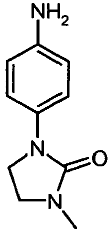

200 mg (1.16 mmol, 1 eq) 4-bromoaniline, 122 mg (1.22 mmol, 1.05 eq) 1-methyl-2-imidazolidinone as well as 22 mg copper(I)-iodide (0.12 mmol, 0.1 eq) are suspended in 2 mL toluene. After the addition of 24.7 µL (0.23 mmol, 0.2 eq) N,N'-dimethylethylenediamine and 321 mg (2.33 mmol, 2 eq) potassium carbonate the reaction mixture is heated in the microwave for 2 h at 140°C with stirring. After cooling, 2 mL MeOH are added, the homogeneous mixture is mixed with water and extracted 5 times with 30 mL EE. The combined organic phases are dried and all the volatile constituents are eliminated in vacuo. Purification by column chromatography (normal phase, silica gel, DCM/MeOH/NH₃ 100/0/ auf 80/20/2) yields 118 mg (0.62 mmol, 53 %) **C-3**.
R_{f} = 0.20 (silica gel, DCM/MeOH/NH₃ 95/5/0.5)
MS-ESI⁺: 192 (M+H)⁺

### C-5) 4-(2-Dimethylamino-ethoxy)-phenylamine

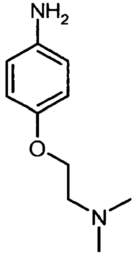

10.2 g (111.7 mmol, 1 eq) 2-dimethylamino-ethanol are dissolved in 320 mL THF and 4.9 g (123 mmol, 1.1 eq, 60 % suspension in oil) sodium hydride are added while cooling with ice. The solution is stirred for 30 min at 4°C and then 15.7 g (109.9 mmol, 1 eq) 4-fluoronitrobenzene, dissolved in 80 mL THF, is added dropwise. The mixture is heated for 2 h to RT and then carefully combined with 50 mL water. Then a total of 350 mL water are added and the mixture is extracted first of all with 400 mL EE and then a further 3 times 200 mL of EE. The combined organic phases and all the volatile constituents are eliminated in vacuo. Purification by column chromatography (normal phase, silica gel, DCM/MeOH 90/10) yields 14.5 g (68.9 mmol, 62 %) dimethyl-[2-(4-nitro-phenoxy)-ethyl]-amine.
MS-ESI⁺: 211 (M+H)⁺
14.4 g (68.5 mmol, 1 eq) dimethyl-[2-(4-nitro-phenoxy)-ethyl]-amine are dissolved in a solvent mixture of 140 mL THF and 20 mL MeOH and combined with 1.25 g palladium on activated charcoal (10 % Pd). The mixture is stirred for 16 h in the autoclave under a hydrogen atmosphere (7 bar). The catalyst is filtered off, washed twice with 25 mL MeOH and the combined organic phases are freed from all volatile constituents in vacuo. 12.3 g (68 mmol, 99%) **C-5** are obtained.
MS-ESI⁺: 181 (M+H)⁺

### C-6) 2-(4-Aminophenyl)-2-methyl-propionic acid

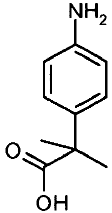

200 mg (0.96 mmol) 2-methyl-2-(4-nitrophenyl)-propionic acid are dissolved in 20 mL MeOH and the solution is combined with 30 mg palladium on activated charcoal (5 % Pd). The mixture is stirred for 16 h at RT in the autoclave under a hydrogen atmosphere (3 bar). The catalyst is filtered off and the filtrate freed from all volatile constituents in vacuo. 171 mg (0.95 mmol, 100 %) **C-6** are obtained, which is used without further purification for the synthesis of **Example 17.**
MS-ESI⁺: 180 (M+H)⁺

### C-7) 2-(4-Aminophenyl)-N-methyl-N-(1-methyl-piperidin-4-yl)-acetamide

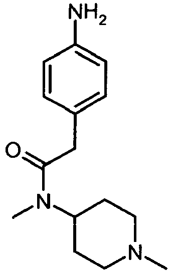

500 mg (2.76 mmol) 4-nitrophenylacetic acid are refluxed in 5 mL thionyl chloride for 2.5 h with stirring, concentrated after cooling in vacuo and the residue is covered with a layer of petroleum ether. During cooling to 0°C and stirring, a precipitate settles out which is filtered off and dried in vacuo. 470 mg (2.36 mmol, 85%) crude 4-nitrophenylacetic acid chloride are obtained.

235 mg (1.18 mmol, 1 eq) of the acid chloride are dissolved in 5 mL DCM and combined with a solution of 297 mg (3.53 mmol, 3 eq) sodium hydrogen carbonate in 5 mL water. The 2-phase system is mixed with 189 µL (1.18 mmol, 1 eq) 1-methyl-4-(methylamino)-piperidine and stirred for 1 h at RT. The organic phase is separated off, the aqueous phase is extracted 3 times with 5 mL DMC, the combined organic phases are dried and all the volatile constituents are eliminated in vacuo. 204 mg (0.70 mmol, 60 %) crude N-methyl-*N*-(1-methyl-piperidin-4-yl)-2-(4-nitro-phenyl)-acetamide are obtained.
MS-ESI⁺: 292 (M+H)⁺

204 mg (0.7 mmol) crude *N*-methyl-*N*-(1-methyl-piperidin-4-yl)-2-(4-nitro-phenyl)-acetamide are dissolved in 4 mL MeOH and this solution is combined with 30 mg palladium on activated charcoal (5 % Pd). The mixture is stirred for 3 days in the autoclave under a hydrogen atmosphere (4 bar), then the catalyst is filtered off and the filtrate is freed from all volatile constituents in vacuo. 198 mg (0.68 mmol, 90%, 97%) **C-7** are obtained, which is used without further purification for the synthesis of Example **18.**

### C-8) 6-Pyrrolidin-1-ylmethyl-pyridin-3-ylamine

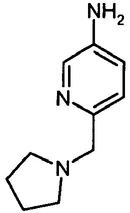

200 mg (1.32 mmol, 1 eq) (5-nitropyridin-2-yl)-acetaldehyde are dissolved in 1 mL DCM, 130 µL (1.58 mmol, 1.2 eq) pyrrolidine are added and the mixture is stirred for 15 min at RT. Then the reaction mixture is combined successively with 90 µL (1.58 mmol, 1.2 eq) acetic acid and 390 mg (1.84 mmol, 1.4 eq) sodium trisacetoxyborohydride. It is stirred for 30 min at RT and the reaction mixture is then combined with 10 mL of saturated, aqueous sodium hydrogen carbonate solution and 20 mL DCM. After phase separation the organic phase is separated off, the aqueous phase is extracted 3 times with 15 mL DCM, the combined organic phases are dried and freed from volatile constituents in vacuo. The crude product (273 mg, 1.32 mmol) is further used directly.
MS-ESI⁺: 208 (M+H)⁺

273 mg (1.32 mmol, 1 eq) 5-nitro-2-pyrrolidin-1-ylmethyl-pyridine are dissolved in 10 mL MeOH, combined with 25 mg palladium on activated charcoal (5 % Pd) and stirred for 2 h in the autoclave under a hydrogen atmosphere (5 bar) at RT. After the catalyst has been filtered off the filtrate is combined with 1 eq dioxanic HCl and all the volatile constituents are eliminated in vacuo. 308 mg crude **C-8** are obtained as the hydrochloride, which is used without further purification in the synthesis of Example **20**.

### C-9) 4-(4-Perhydroazepin-1-yl-piperidin-1-yl)-phenylamine

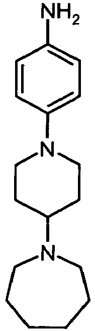

1 g (5 mmol, 1 eq) 1-Boc-piperidin-4-one is dissolved in 15 mL 1,2-dichloroethane and 566 µL (5 mmol, 1 eq) perhydroazepine are added. The mixture is stirred for 15 min at RT and then 0.29 mL (5 mmol, 1 eq) acetic acid as well as 1.57 g (7 mmol, 1.4 eq) sodium trisacetoxyborohydride are added. The mixture is stirred for 16 h at RT and the reaction mixture is then combined with saturated, aqueous sodium hydrogen carbonate solution (50 mL). It is extracted twice with 50 mL DCM, the combined organic phases are dried and after the elimination of all the volatile constituents in vacuo, 1.23 g (4.4 mmol, 87 %) crude *tert*-butyl 4-perhydroazepin-1-yl-piperidine-1-carboxylate is obtained, which is reacted further directly.

1.23 g (4.4 mmol) *tert*-butyl 4-perhydroazepin-1-yl-piperidine-1-carboxylate are dissolved in 4 mL diethyl ether and the solution is combined with 8 mL (6.3 eq, 4 M in dioxane) hydrochloric acid. After 16 h stirring at RT all the volatile constituents are eliminated in vacuo and the residue is washed with diethyl ether. After drying in vacuo 1.04 g of crude 1-piperidin-4-yl-perhydroazepine hydrochloride are obtained, which are used without further purification.

80 µL (0.75 mmol, 1 eq) 4-fluoronitrobenzene are dissolved in 0.5 mL isopropanol, and combined with 248 mg (1.13 mmol, 1.5 eq) 1-piperidin-4-yl-perhydroazepine hydrochloride and 368 µL (2.64 mmol, 3.5 eq) triethylamine. The reaction mixture is then heated for 30 min at 160°C in the microwave with stirring and after cooling poured into 50 mL water. It is made basic with conc. aqueous ammonia solution, extracted 3 times with 50 mL EE and the combined organic phases are dried. After all the volatile constituents have been eliminated in vacuo, 393 mg (content 58 %) of crude 1-[1-(4-nitro-phenyl)-piperidin-4-yl]-perhydroazepine is obtained, which is used in the next step without further purification.
MS-ESI⁺: 304 (M+H)⁺

393 mg (0.75 mmol, 58 %) 1-[1-(4-nitro-phenyl)-piperidin-4-yl]-perhydroazepine is dissolved in 5 mL THF and a spatula tip of Raney nickel is added. The mixture is stirred for 16 h at RT in the autoclave under a hydrogen atmosphere (5 bar), the catalyst is filtered off, the filtrate is combined with 0.8 mL 4 M hydrochloric acid in dioxane and concentrated in vacuo. 294 mg (0.76 mmol, 100%) of crude **C-9** are obtained.

The components 1-(4-amino-phenyl)-piperidin-4-yl]-cyclopentylamine **(C-10)** and 4-(4-morpholin-4-yl-piperidin-1-yl)-phenylamine **(C-11)** may be prepared analogously, and are used as starting compounds for the synthesis of Examples 22 and 23.

### C-12) tert. Butyl (S)-3-(4-aminophenoxy)-pyrrolidine-1-carboxylate

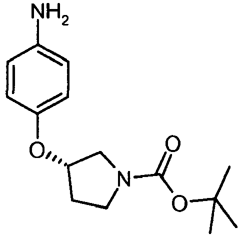

3.1 g (16.56 mmol, 1.18 eq) tert. butyl (S)-3-hydroxy-pyrrolidin-1-carboxylate are dissolved in 80 mL THF and 900 mg (22.5 mmol, 1.6 eq, 60 % dispersion in oil) of sodium hydride are added while cooling with ice to 5°C. The mixture is stirred for 15 min at 5°C and then 1.5 mL (14 mmol, 1 eq) 4-fluoronitrobenzene, dissolved in 20 mL THF, are slowly added dropwise. The mixture is heated to RT and stirred for 5 h at RT. Then 50 mL water are added, the mixture is stirred again and finally the reaction mixture is poured into 200 mL water and extracted 3 times with EE. The combined organic phases are dried and all the volatile constituents are eliminated in vacuo. Purification by column

chromatography (normal phase, silica gel, cHex/EE 9/1 to 8/2) yields 3 g (9.7 mmol, 69 %) tert-butyl (S)-3-(4-nitro-phenoxy)-pyrrolidine-1-carboxylate. 3 g (9.7 mmol) tert. butyl (S)-3-(4-nitro-phenoxy)-pyrrolidine-1-carboxylate are dissolved in 100 mL THF, the reaction mixture is combined with 310 mg palladium on activated charcoal (5 % Pd) and stirred for 16 h at RT in an autoclave under a hydrogen atmosphere (6 bar). After the catalyst has been filtered off all the volatile constituents are eliminated in vacuo and 2.8 g of crude **C-12** are obtained, which is reacted without further purification in the syntheses of Examples **24, 25** and **29.**

Tert. butyl (R)-3-(4-amino-phenoxy)-pyrrolidine-1-carboxylate **C-13** is obtained analogously starting from tert. butyl (R)-3-hydroxy-pyrrolidin-1-carboxylate. **C-13** is used as starting material for the synthesis of Examples **26 - 28.**

### C-14) tert. Butyl (R)-3-(3-aminophenylamino)-pyrrolidine-1-carboxylate

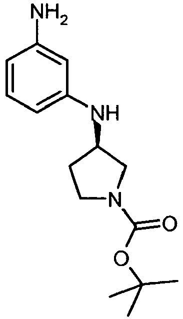

1.15 g (6.17 mmol, 1 eq) tert. butyl (R)-3-amino-pyrrolidine-1-carboxylate are dissolved in 5.8 mL DMSO and then 20 g (145 mmol, 23.4 eq) potassium carbonate are added. Then 657 µL (6.17 mmol, 1 eq) 4-fluoronitrobenzene are added dropwise. The reaction mixture is stirred for 8 days at 140°C, while potassium carbonate is added from time to time (total 20 g). It is thawed to RT and the reaction mixture is finally poured into 150 mL water and extracted 3 times with 50 mL EE. The combined organic phases are washed with saturated aqueous sodium chloride solution, dried and all the volatile constituents are eliminated in vacuo. 2.2 g (4.2 mmol, 68 %) tert. butyl (R)-3-(3-nitro-phenylamino)-pyrrolidine-1-carboxylate are obtained.
MS-ESI⁺: 252 (M+H)⁺

2.16 g (4.2 mmol) tert. butyl (R)-3-(3-nitrophenylamino)-pyrrolidine-1-carboxylate are dissolved in 32 mL MeOH, the reaction mixture is combined with 131 mg Raney nickel and stirred at RT in an autoclave under a hydrogen atmosphere (6 bar) for 16 h. After the catalyst has been filtered off all the volatile constituents are eliminated in vacuo and 1.15 g of crude **C-14** are obtained, which is reacted without further purification in the syntheses of Examples **30, 31** and **32.**
MS-ESI⁺: 278 (M+H)⁺

### C-15) tert. Butyl (S)-3-(4-aminophenylamino)-pyrrolidine-1-carboxylate

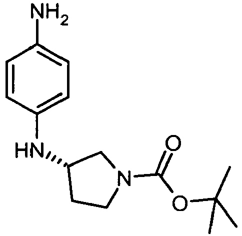

### C-16) tert. Butyl (R)-3-(4-amino-phenylamino)-pyrrolidine-1-carboxylate

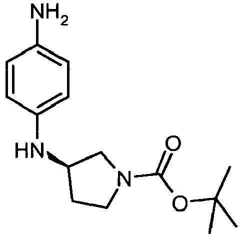

**C-15** and **C-16** are prepared analogously to **C-14,** using DMSO as solvent and triethylamine (3.5 eq) as base. The reaction temperature is 100 - 120°C and the reaction lasts 5 - 16 h. The reductions of the corresponding nitro intermediates are carried out in THF using palladium on activated charcoal.

Using 4-fluoro-3-methoxy-nitrobenzene, 3-chloro-4-fluoro-nitrobenzene, 3,4-difluoronitrobenzene or 2-fluoro-5-nitrotoluene the substituted synthesis components for preparing **Examples 37 - 41** are synthesised analogously to the procedure described for **C-14.**

### D-1) Benzyl 4-aminobenzoate

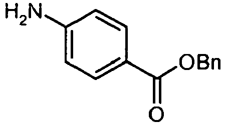

15.01 g (89.9 mmol) 4-nitrobenzoic acid are suspended in 500 mL MeCN and then mixed with 15.03 g (108.7 mmol, 1.2 eq) potassium carbonate. 15.4 g (90.4 mmol, 1.01 eq) benzylbromide are added dropwise with stirring and the reaction mixture is stirred for 5 h at 60°C. It is combined with 750 mL distilled water, extracted 4 times with 250 mL of EE and after the organic phases have been combined they are dried on sodium sulphate. After all the volatile constituents have been eliminated in vacuo the crude product is suspended twice in toluene in succession and all the volatile constituents are eliminated in vacuo. 20.6 g (80.1 mmol) benzyl 4-nitrobenzoate are obtained, which is used in the next step without further purification.

20.6 g of the benzyl 4-nitrobenzoate are dissolved in 350 mL dioxane and this solution is combined with 6.9 g (49.9 mmol, 0.61 eq) Raney nickel. The mixture is hydrogenated for 16 h with stirring at 5 bar of H₂ pressure. The catalyst is filtered off, all the volatile constituents are eliminated in vacuo and 17 g of **D-1** are obtained.

### D-2) Benzyl 4-(4-chloro-5-trifluoromethylpyrimidin-2-ylamino)-benzoate

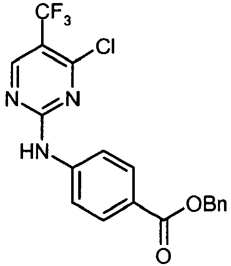

10 g (44 mmol) benzyl 4-aminobenzoate **D-1** are dissolved in 200 mL DMA, 8 mL of Hünig base (0.97 eq) are added and 10.4 g (48.21 mmol) of 2,4-dichloro-5-trifluoromethylpyrimidine, dissolved in 50 mL DMA, are added dropwise to this solution at RT. The reaction solution is stirred overnight at 60°C, then combined with 300 mL DCM and extracted with distilled water (3 times 300 mL). The organic phase is dried and the solvent is eliminated in vacuo. The crude product is combined with 100 mL MeOH, digested and left to stand for 2 h. Then it is stirred for 10 min, the precipitate is filtered off and washed with MeOH. Finally the crude product is suspended again in MeOH, filtered off, washed with a little MeOH and dried at 60°C in the vacuum dryer. 8.5 g of **D-2** are obtained.
R_{f} = 0.71 (silica gel, cHex:EE 1:2)
MS-ESI⁺: 408 (M+H)⁺

### D-3) Benzyl 4-[4-((1R,2S)-2-carboxycyclopentylamino)-5-trifluoromethyl-pyrimidin-2-ylamino]-benzoate

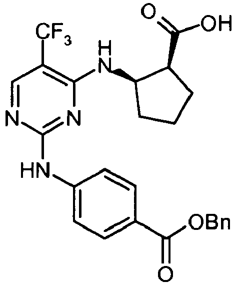

2.05 g (5 mmol, 1 eq) of **D-2** and 1 g (1S,2R)-2-amino-1-cyclopentanecarboxylic acid hydrochloride (6 mmol, 1.2 eq) are placed in 18 mL EtOH, 7.3 mL (42.5 mmol, 8.5 eq) Hünig base are added and the mixture is stirred for 4 h at 70°C. The reaction mixture is stirred into 275 mL water, filtered to remove the insoluble matter, the filtrate is adjusted to pH 2 with saturated, aqueous KHSO₄ solution, stirred for 5 min and the resulting precipitation is suction filtered. The crude product is washed with water, dried in vacuo and 2.37 g **D-3** are obtained.
MS-ESI⁺: 501 (M+H)⁺

### D-4) Benzyl 4-[4-((1R,2S)-2-isopropylcarbamoylcyclopentylamino)-5-trifluoromethylpyrimidin-2-ylamino]-benzoate

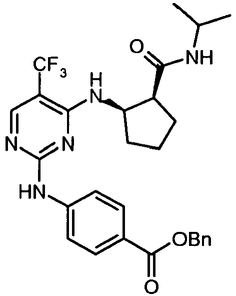

2.59 g (4.9 mmol) **D-3**, 2.21 g (6.9 mmol, 1.4 eq) TBTU and 4.21 mL (24.6 mmol, 5 eq) Hünig base are dissolved in 75 mL DMF and stirred for 20 min at RT. Then 0.63 mL (7.38 mmol, 1.5 eq) isopropylamine are added and the mixture is stirred overnight at RT. It is suction filtered through basic aluminium oxide, washed with DMF and the mother liquor is stirred into 400 mL water, stirred for another 30 min and the precipitation is suction filtered. The crude product is washed with water and dried in vacuo. For purification it is stirred with 50 mL MeCN for 30 min at 5°C, suction filtered, washed with a little cold MeCN and the residue is dried in vacuo. 2.13 g **D-4** are obtained.
R_{f} = 0.53 (silica gel, cHex:EE 1:1)
MS-ESI⁺: 542 (M+H)⁺

### D-5) 4-[4-((1R,2S)-2-Isopropylcarbamoylcyclopentylamino)-5-trifluoromethylpyrimidin-2-ylamino]-benzoic acid

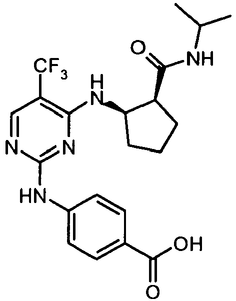

2.13 g (3.9 mmol) **D-4** are dissolved in 150 mL THF and 250 mg palladium hydroxide/C catalyst (20 wt.% Pd on activated charcoal) are added. The mixture is hydrogenated for 16 h at a H₂ pressure of 6 bar with stirring at RT. Then 30 mL MeOH are added, the catalyst is filtered through kieselguhr, washed with MeOH and the filtrate is evaporated down. The residue is decocted with 45 mL EtOH, slowly cooled to 5°C, stirred for another 1 h and then suction filtered and washed with cold EtOH. 2.46 g **D-5** are obtained.
R_{f} = 0.46 (silica gel, CH₂Cl₂:MeOH:AcOH 5:1:0.1)
MS-ESI⁺: 452 (M+H)⁺

### Preparation of the Examples

### Example 1

### (1S,2R)-2-[5-Bromo-2-(4-dimethylaminomethyl-phenylamino)-pyrimidin-4-ylamino]-cyclopentanecarboxylic acid-isopropylamide

250 mg (0.69 mmol) **A**-2d are dissolved in 1.5 mL 1-butanol, 650 µL (2.6 mmol, 3.8 eq) hydrochloric acid (4 M in dioxane) and 290 mg (1.74 mmol, 2.5 eq) **C**-1 are added. The reaction mixture is heated for 45 min at 130°C in the microwave with stirring. After cooling the precipitate is filtered off and the filtrate is combined with RP gel. The volatile constituents are eliminated in vacuo and the product is purified and isolated by column chromatography through an RP phase (water/MeCN (+ in each case 0.2% HCOOH) from 86/14 to 69/31 in 15 min). Corresponding product fractions are combined, mixed with concentrated hydrochloric acid, freed from the solvent by freeze-drying and 75 mg of compound **1** are obtained as the hydrochloride.
RT = 2.04 min
MS-ESI⁺: 475/477 (M+H)⁺

Examples **2** and **6** are prepared analogously, and as described in the general **synthesis scheme A,** using MeOH as solvent. In the case of Example **2** the starting material is component **A-2c,** while the synthesis of Example **6** starts from component **A-2e** accordingly.

### Example 3

### (1S,2R)-2-[2-(4-Pyrrolidin-1-ylmethyl-phenylamino)-5-trifluoromethyl-pyrimidin-4-ylamino]-cyclopentanecarboxylic acid-isopropylamide

The synthesis is carried out starting from **D-5** in a reduction-oxidation sequence followed by reductive amination.

885 mg (1.96 mmol, 1.0 eq) of **D-5** are suspended in 200 mL THF under protective gas at 0°C and 19.6 mL (19.6 mmol, 10 eq) borane-THF complex (1 M solution in THF) is added dropwise to the suspension at 0°C. The mixture is stirred for 1 h at 0°C and 2 h at RT and then slowly combined with 200 mL water. The precipitate is filtered off and dried in vacuo. 689 mg (1.6 mmol, 80%) crude (1S,2R)-2-[2-(4-hydroxymethyl-phenylamino)-5-trifluoromethyl-pyrimidin-4-ylamino]-cyclopentanecarboxylic acid-isopropylamide are obtained, which is used without further purification.

689 mg (1.58 mmol, 1 eq) (1S,2R)-2-[2-(4-hydroxymethyl-phenylamino)-5-trifluoro-methylpyrimidin-4-ylamino]-cyclopentanecarboxylic acid-isopropylamide are dissolved in 30 mL THF, and 2.5 mg (0.016 mmol, 0.01 eq) TEMPO and 0.42 g (1.73 mmol 1.1 eq) trichloroisocyanuric acid are added successively to this solution at 0°C. It is stirred for 30 min at 0°C and for a further 2 h at RT until HPLC monitoring of the reaction indicates total conversion. The solution is combined with 150 mL DCM and then washed 3 times with 50 mL water. The organic phase is dried on magnesium sulphate, all the volatile constituents are eliminated in vacuo and 720 mg of crude (1S,2R)-2-[2-(4-formyl-phenylamino)-5-trifluoromethyl-pyrimidin-4-ylamino]-cyclopentane-carboxylic acid isopropylamide are obtained, this substance being used without further purification in the reductive amination.

720 mg (1.65 mmol, 1 eq) (1S,2R)-2-[2-(4-formyl-phenylamino)-5-trifluoromethyl-pyrimidin-4-ylamino]-cyclopentanecarboxylic acid-isopropylamide are dissolved in 5 mL DMF, 273 µL (3.3 mol, 2 eq) pyrrolidine are added and the mixture is stirred for 10 min at RT. Then 113 µL (2.0 mmol, 1.2 eq) acetic acid and 526 mg (2.5 mmol, 1.5 eq) sodium trisacetoxyborohydride are added and the reaction mixture is stirred for 1 h at RT. The reaction mixture is directly combined with RP gel, the volatile constituents are eliminated in vacuo and the product is purified by column chromatography (water/MeCN 95/5 (+ in **each** case 0.2% HCOOH) to 2/98 in 15 min). Corresponding product fractions are combined, mixed with concentrated hydrochloric acid, freed from the solvent by freeze-drying and 100 mg of compound **3** are obtained as the hydrochloride.
RT = 1.50 min
MS-ESI⁺: 491 (M+H)⁺

Examples **4** and **5** are prepared analogously.

### Example 7

### (1S,2R)-2-[5-Bromo-2-(3-morpholin-4-ylmethylphenylamino)-pyrimidin-4-ylamino]-cyclopentanecarboxylic acid-isopropylamide

631 mg (1.74 mmol, 1 eq) **A-2d** are suspended in 3 mL DMA and 465 mg (3.84 mmol, 2.21 eq) 3-aminobenzaldehyde are added. After the addition of 1.08 mL (4.32 mmol, 2.48 eq, 4 M in dioxane) hydrochloric acid the reaction mixture is heated for 24 h at 70°C. The reaction mixture is poured onto 100 mL water, the precipitate formed is filtered off and dried in vacuo. 450 mg (1.01 mmol, 58%) crude (1S,2R)-2-[5-bromo-2-(3-formyl-phenylamino)-pyrimidin-4-ylamino]-cyclopentanecarboxylic acid isopropylamide are obtained, which is used without further purification.

70 mg (0.16 mmol, 1 eq) (1S,2R)-2-[5-bromo-2-(3-formylphenylamino)-pyrimidin-4-ylamino]-cyclopentanecarboxylic acid-isopropylamide are dissolved in 1.7 mL DMA and combined with 51 µL (0.47 mmol, 3 eq) morpholine. The solution is stirred for 30 min at RT and then 27 µL (0.47 mmol, 3 eq) acetic acid and 31 mg (0.47 mmol, 3 eq) sodium trisacetoxyborohydride are added. After 16 h stirring at RT the mixture is purified by chromatography through a RP phase (MeCN/water 5/95 + (in each case 0.2% HCOOH) to 25/75 in 12 min). After the product fractions have been combined, hydrochloric acid (4 M in dioxane) has been added and the mixture has been freeze-dried, 8 mg of the hydrochloride of compound 7 is obtained.
RT = 1.94 min
MS-ESI⁺: 517/519 (M+H)⁺

Examples **8** and **9** are prepared analogously using pyrrolidine or 4-amino-1-methylpiperidine.

### Example 10

### (1S,2R)-2-(5-Chloro-2-{4-[4-(4-isobutyl-piperazin-1-yl)-piperidin-1-yl]-phenylamino}-pyrimidin-4-ylamino)-cyclopentanecarboxylic acid-isopropylamide

804 mg (2.54 mmol, 1 eq) **A-2c** are dissolved in 10 mL MeOH, combined with 905 mg (2.51 mmol, 1 eq) **C-2** and after the addition of 127 µL hydrochloric acid (0.51 mmol, 0.2 eq, 4 M in dioxane) the reaction mixture is heated in the microwave with stirring for 30 min at 130°C. After cooling the reaction mixture is combined with silica gel and after all the volatile constituents have been eliminated in vacuo, 591 mg (1.1 mmol, 44 %) (1S,2R)-2-{5-chloro-2-[4-(4-piperazin-1-yl-piperidin-1-yl)-phenylamino]-pyrimidin-4-ylamino}-cyclopentanecarboxylic acid-isopropylamide are obtained by purification by column chromatography (normal phase, silica gel, DCM/MeOH from (80/20 to 40/60). MS-ESI⁺: 541 (M+H)⁺

50 mg (0.09 mmol, 1 eq) (1S,2R)-2-{5-chloro-2-[4-(4-piperazin-1-yl-piperidin-1-yl)-phenylamino]-pyrimidin-4-ylamino}-cyclopentanecarboxylic acid-isopropylamide are dissolved in 200 µL DMF and after the addition of 16.7 µL (0.18 mmol, 2 eq) isobutyraldehyde the reaction mixture is stirred for 1 h at RT. Then 5 µL (0.09 mmol, 1 eq) acetic acid and 19.5 mg (0.09 mmol, 1 eq) sodium trisacetoxyborohydride (dissolved in a little DMF) are added successively and the mixture is stirred for 16 h at RT. It is purified by chromatography through an RP phase. After the product fractions have been combined, conc. hydrochloric acid has been added (4 M in dioxane) and the mixture has been freeze-dried, 24 mg of the hydrochloride of compound **10** are obtained.
RT = 2.32 min
MS-ESI⁺: 597 (M+H)⁺

Examples **11** and **12** are prepared analogously using formaldehyde or cyclopropanecarbaldehyde.

### Example 13

### (1S,2R)-2-{2-[4-(3-Methyl-2-oxo-imidazolidin-1-yl)-phenylamino]-5-trifluoromethyl-pyrimidin-4-ylamino}-cyclopentanecarboxylic acid-isopropylamide

50 mg (0.14 mmol, 1 eq) **A-2a** are suspended with 41 mg (0.21 mmol, 1.5 eq) **C-3** in a little NMP and the reaction mixture is combined with 7 µL (0.03 mmol, 0.2 eq, 4 M in dioxane) hydrochloric acid. It is heated in the microwave for 30 min at 100°C with stirring, then the reaction mixture is diluted with MeOH, poured into 25 mL water and concentrated in vacuo. The precipitate is filtered off and dried in vacuo. After the addition of 1 eq HCl (aq) the mixture is lyophilised and 66 mg (0.12 mmol, 85%) of the hydrochloride of compound **13** are obtained.
RT = 1.82 min
MS-ESI⁺: 506 (M+H)⁺

### Example 15

### (1S,2R)-2-{5-Bromo-2-[4-(2-dimethylamino-ethoxy)-phenylamino]-pyrimidin-4-ylamino}-cyclopentanecarboxylic acid-isopropylamide

356 mg (0.98 mmol, 1 eq) **A-2d** are dissolved in a mixture of 9 mL MeOH and 1.8 mL water, then 197 mg (1.08 mmol, 1.1 eq) **C-5** are added and finally the reaction mixture is combined with 145 µL (1.91 mmol, 1.9 eq) of trifluoroacetic acid. The reaction mixture is heated for 16 h at 105°C with stirring (microwave), then after cooling to RT concentrated in vacuo and combined with 25 mL aqueous conc. ammonia solution. The precipitate formed is filtered off and after being taken up in a little water/MeCN (1/3) it is purified by column chromatography through an RP phase. Corresponding product fractions are combined, freed from the solvent by freeze-drying and 301 mg of compound **15** are obtained.
RT = 1.99 min
MS-ESI⁺: 505/507 (M+H)⁺

### Example 16

### Methyl {4-[4-((1R,2S)-2-isopropylcarbamoyl-cyclopentylamino)-5-trifluoromethyl-pyrimidin-2-ylamino]-phenyl}-acetate

100 mg (0.29 mmol, 1 eq) **A-2a** are dissolved in 2 mL 1-butanol, 66 mg (0.34 mmol, 1.2 eq, 85%) methyl 4-aminophenyl-acetate are added and after the addition of 21 µL (0.09 mmol, 0.3 eq, 4 M in dioxane) hydrochloric acid the mixture is heated for 2 h to 70°C with stirring. The reaction mixture is combined directly with RP gel, the volatile constituents are eliminated in vacuo and the product is purified by column chromatography through an RP phase (water/MeCN 70/30 (+ in each case 0.2% HCOOH) to 40/60 in 15 min). Corresponding product fractions are combined, mixed with conc. aqueous hydrochloric acid and freed from the solvent by freeze-drying. 56 mg of compound **16** are obtained.
RT = 2.09 min
MS-ESI⁺: 480 (M+H)⁺

Examples **17** and **18** are prepared under analogous reaction conditions and using **C-6** and **C-7.**

### Example 19

### Methyl 5-[4-((1R,2S)-2-isopropylcarbamoyl-cyclopentylamino)-5-trifluoromethyl-pyrimidin-2-ylaminol-pyridine-2-carboxylate-(1-methyl-piperidin-4-yl)-amide

300 mg (0.86 mmol, 1 eq) **A-2a** are dissolved in 1 mL DMA, 154 mg (1.11 mmol, 1.3 eq) 5-aminopyridine-2-carboxylic acid are added and the reaction mixture is then combined with 492 µL (1.97 mmol, 2.3 eq, 4 M in dioxane) hydrochloric acid. The reaction mixture is stirred for 48 h at 70°C, combined directly with RP gel, the volatile constituents are eliminated in vacuo and the product is purified by column chromatography (water/MeCN 78/22 (+ in each case 0.2% HCOOH) to 60/40 in 15 min). Corresponding product fractions are combined and freed from the solvent by freeze-drying. 11 mg of 5-[4-((1R,2S)-2-isopropylcarbamoyl-cyclopentylamino)-5-trifluoromethyl-pyrimidin-2-ylamino]-pyridine-2-carboxylic acid are obtained.
MS-ESI⁺: 453 (M+H)⁺

11 mg (24 µmol, 1 eq) 5-[4-((1R,2S)-2-isopropylcarbamoyl-cyclopentylamino)-5-trifluoromethyl-pyrimidin-2-ylamino]-pyridine-2-carboxylic acid are dissolved in 120 µL DMF, then first of all 21 µL (0.12 mmol, 4.9 eq) Hünig base and then 11 mg (34 µmol, 1.4 eq) TBTU are added. The reaction mixture is stirred for 20 min at RT and then combined with 5 µL (36 µmol, 1.5 eq) 1-methyl-4-(methylamino)-piperidine. The mixture is stirred for 16 h at RT, then combined directly with RP gel, the volatile constituents are eliminated in vacuo and the product is purified by column chromatography (water/MeCN 88/12 (+ in each case 0.2% HCOOH) to 70/30 in 15 min). Corresponding product fractions are combined, mixed with dioxanic hydrochloric acid and freed from the solvent by freeze-drying. 8 mg of the hydrochloride of compound **19** are obtained.
RT = 1.80 min
MS-ESI⁺: 563 (M+H)⁺

### Example 20

### (1S,2R)-2-[2-(6-Pyrrolidin-1-ylmethyl-pyridin-3-ylamino)-5-trifluoromethyl-pyrimidin-4-ylamino]-cyclopentanecarboxylic acid-isopropylamide

40 mg (114 µmol, 1 eq) **A-2a** are dissolved in 0.4 mL NMP, 37 mg (171 µmol, 1.5 eq) **C-8** are added, combined with 14 µL (0.06 mmol, 0.5 eq, 4 M in dioxane) hydrochloric acid and heated to 120°C in the microwave for 1 h with stirring. The reaction mixture is then purified directly by chromatography through an RP phase (water/MeCN (+ in each case 0.2% HCOOH) from 5/95 to 50/50). After combining the product fractions, adding hydrochloric acid (4 M in dioxane) and freeze-drying, 40 mg of the hydrochloride of compound **20** is obtained.
RT = 2.00 min
MS-ESI⁺: 492 (M+H)⁺

### Example 21

### (1S,2R)-2-{2-[4-(4-Perhydro-azepin-1-yl-piperidin-1-yl)-phenylamino]-5-trifluoromethyl-pyrimidin-4-ylamino}-cyclopentanecarboxylic acid-isopropylamide

77 mg (0.22 mmol, 1 eq) **A-2a** are dissolved in 1.7 mL NMP, 80 mg (0.21 mmol, 0.95 eq) **C-9** are added and the reaction mixture is heated to 100°C for 16 h with agitation. The reaction mixture is then purified directly by chromatography through an RP phase (water/MeCN (+ in each case 0.2% HCOOH) from 85/15 to 65/35 in 15 min). After combining the product fractions, adding 0.4 mL hydrochloric acid (4 M in dioxane) and freeze-drying, 88 mg of the bis-hydrochloride of compound **21** is obtained.
RT = 2.65 min
MS-ESI⁺: 588 (M+H)⁺

Examples **22** and **23** are prepared analogously starting from **A-2d** and **C-10** and also starting from **A-2a** and **C-11.** In both cases, some Hünig base is added in order to bind excess hydrochloric acid from the hydrochlorides **C-10** and **C-11** used.

### Example 24

### (11S,2R)-2-(5-Bromo-2-{4-[(S)-1-(tetrahydro-pyran-4-yl)-pyrrolidin-3-yloxy]-phenylamino}-pylimidin-4-ylamino)-cyclopentanecarboxylic acid-isopropylamide

1.123 g (3.11 mmol, 1 eq) **A-2d** are dissolved in 12 mL MeOH, 1.198 g (4.27 mmol, 1.4 eq) **C-12** and then 1.22 ml (4.87 mmol, 1.6 eq, 4 M in dioxane) hydrochloric acid are added. The reaction mixture is heated to 130°C for 40 min in the microwave with stirring. After cooling the reaction mixture is poured into 200 mL water, made basic with potassium hydroxide and the precipitate is filtered off. It is washed with MeOH and dried in vacuo. 1.70 g of crude (1S,2R)-2-{5-bromo-2-[4-((S)-pyrrolidin-3-yloxy)-phenylamino]-pyrimidin-4-ylamino}-cyclopentane-carboxylic acid-isopropylamide are obtained, which are reacted without further purification. 79 mg (157 µmol, 1 eq) (1S,2R)-2-{5-bromo-2-[4-((S)-pyrrolidin-3-yloxy)-phenylamino]-pyrimidin-4-ylamino}-cyclopentanecarboxylic acid-isopropylamide are dissolved in 1.5 mL DMA, 19 mg (188 µmol, 1.2 eq) tetrahydro-4H-pyran-4-one are added, followed by 22 µL (377 µmol, 2.4 eq) acetic acid. The reaction mixture is stirred for 30 min at RT and then 107 mg (507 µmol, 3.2 eq) sodium trisacetoxyborohydride are added. Then the reaction mixture is stirred for 2 h at RT and then purified directly by chromatography through an RP phase (water/MeCN (+ in each case 0.2% HCOOH) from 95/5 to 65/35 in 10 min). After combining the product fractions, adding 0.2 mL hydrochloric acid (4 M in dioxane) and freeze-drying, 83 mg of the hydrochloride of compound **24** is obtained.
RT = 1.98 min
MS-ESI⁺: 587/589 (M+H)⁺

Examples **25, 26** and **28** are prepared analogously by reacting the components **A-2d** or **A-2a** and **C-12** or **C-13** and subsequent reductive amination with the corresponding aldehydes.

### Example 27

### (1S,2R)-2-(5-Bromo-2-{4-[(R)-1-(2-fluoro-ethyl)-pyrrolidin-3-yloxy]-phenylamino}-pyrimidin-4-ylamino)-cyclopentanecarboxylic acid-isopropylamide

146 mg (290 µmol, 1 eq) (1S,2R)-2-{5-bromo-2-[4-((R)-pyrrolidin-3-yloxy)-phenylamino]-pyrimidin-4-ylamino}-cyclopentanecarboxylic acid-isopropylamide (prepared in the same way as the enantiomeric component, described in Example **24**) are dissolved in 1 mL DMF and 77 mg (556 µmol, 1.9 eq) potassium carbonate are added. After the addition of 24 mg (191 µmol, 0.7 eq) 1-bromo-2-fluoroethane the mixture is stirred for 20 h at RT. The reaction mixture is then purified directly by chromatography through an RP phase (water/MeCN (+ in each case 0.2 % HCOOH) from 95/5 to 63/37 in 10 min). After combining the product fractions, adding 0.2 mL hydrochloric acid (4 M in dioxane) and freeze-drying, 64 mg of the hydrochloride of **27** is obtained.
RT = 2.01 min
MS-ESI⁺: 549/551 (M+H)⁺

Example **29** is obtained analogously, starting from **A-2b** and **C-12** and then carrying out alkylation as described for Example **27.**

Example **31** is obtained analogously to the method described in Example **27,** starting from **A-2d** and **C-14** and carrying out alkylation as described for Example **27.**

Examples **30** and **32** are obtained analogously to the method described in Example **24,** starting from **A-2d** or **A-2a** and **C-14** and carrying out reductive amination as described for Example **24.**

Examples **33 - 36** are obtained analogously to the method described in Example **24** or Example **27,** starting from **A-2a, A-2c** or **A-2d** and **C-15** or **C-16.** In the case of Example **33** and **35** alkylation is carried out as described for Example **27** and in the case of Example **34** and **36** reductive amination is carried out as described for Example **24.**

Examples **37 - 41** are obtained analogously to the method described in Example **24** or Example **27,** using **A-2b, A-2c** or **A-2d** and the substituted synthesis components prepared analogously to **C-14, C-15** and **C-16** (see the preparation of the synthesis components C).

### Examples 1 - 41

| **Ex. no.** | **Structure** | **method of synthesis** | **HPLC RT [min]** | **MS (ESI⁺) [M+H]⁺** | **UVₘₐₓ [nm]** |
|---|---|---|---|---|---|
| 1 | 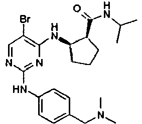 | A | 2.04 | 475/477 | 270 |
| 2 | 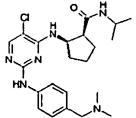 | A | 2.00 | 431 | 270 |
| 3 | 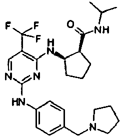 | D | 1.50 | 491 | 270 |
| 4 | 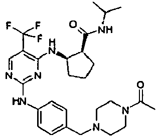 | D | 1.45 | 548 | 274 |
| 5 | 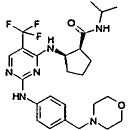 | D | 1.42 | 507 | 274 |
| 6 | 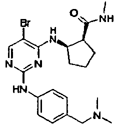 | A | | 447/449 | 270 |
| 7 | 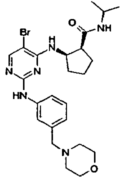 | A | 1.94 | 517/519 | 266 |
| 8 | 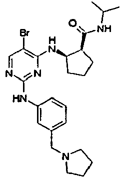 | A | 2.25 | 501/503 | 266 |
| 9 | 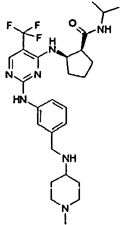 | A | 2.21 | 534 | 266 |
| 10 | 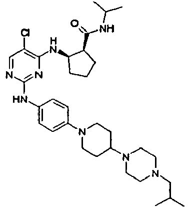 | A | 2.32 | 597 | 274 |
| 11 | 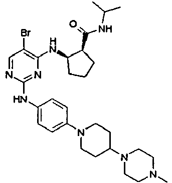 | A | 1.90 | 599/601 | 272 |
| 12 | 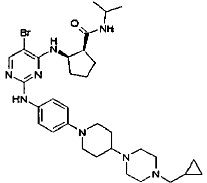 | A | 2.12 | 639/641 | 274 |
| 13 | 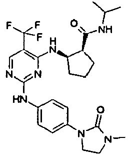 | A | 1.82 | 506 | 282 |
| 15 | 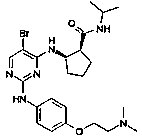 | A | 1.99 | 505/507 | 266 |
| 16 | 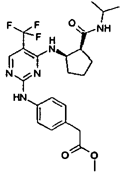 | A | 2.09 | 480 | 266 |
| 17 | 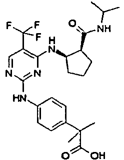 | A | 1.38 | 494 | 270 |
| 18 | 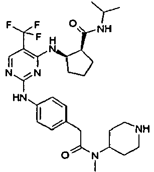 | A | 1.94 | 562 | 237/294 |
| 19 | 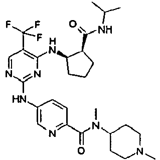 | A | 1.80 | 563 | 248/272/ 301 |
| 20 | 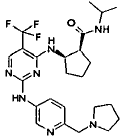 | A | 2.0 | 492 | 248/270/ 294 |
| 21 | 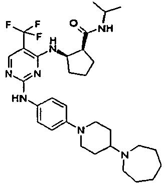 | A | 2.65 | 588 | 273 |
| 22 | 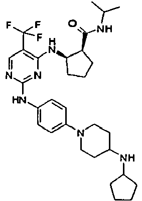 | A | 2.44 | 584/586 | 277 |
| 23 | 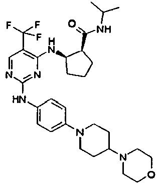 | A | 1.99 | 576 | 274 |
| 24 | 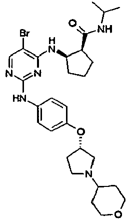 | A | 1.98 | 587/589 | 266 |
| 25 | 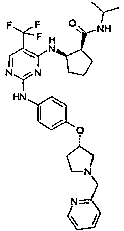 | A | 2.10 | 584 | 230/258 |
| 26 | 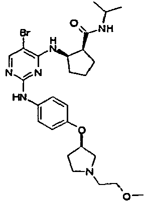 | A | 2.00 | 561/563 | 266 |
| 27 | 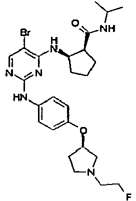 | A | 2.01 | 549/551 | 266 |
| 28 | 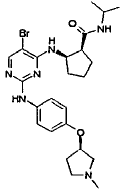 | A | 2.03 | 517/519 | 266 |
| 29 | 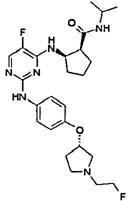 | A | 1.86 | 489 | 262 |
| 30 | 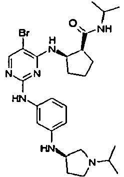 | A | 2.22 | 544/546 | 246 |
| 31 | 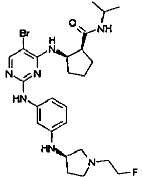 | A | 1.97 | 548/550 | 230 |
| 32 | 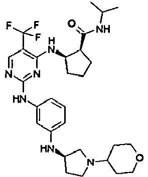 | A | 2.05 | 576 | 238 |
| 33 | 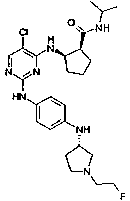 | A | 1.87 | 504 | 274 |
| 34 | 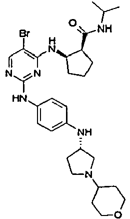 | A | 1.89 | 586/588 | 274 |
| 35 | 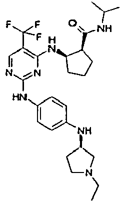 | A | 2.18 | 520 | 226 |
| 36 | 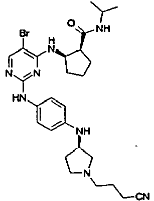 | A | 1.92 | 569/571 | 226/272 |
| 37 | 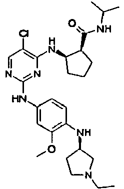 | A | 2.13 | 516 | 276 |
| 38 | 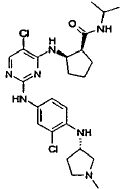 | A | 2.14 | 506 | 278 |
| 39 | 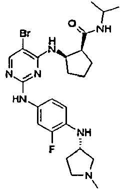 | A | 2.06 | 534/536 | 278 |
| 40 | 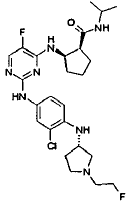 | A | 1.97 | 522 | 270 |
| 41 | 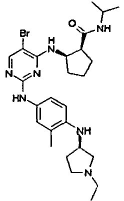 | A | 2.17 | 544/546 | 286 |

The Examples describe the biological activity of the compounds according to the invention without restricting the invention to these Examples.

As demonstrated by DNA staining followed by FACS or Cellomics Array Scan analysis, the inhibition of proliferation brought about by the compounds according to the invention is mediated above all by errors in chromosome segregation. Because of the accumulation of faulty segregations, massive polyploidy occurs which may finally lead to inhibition of proliferation or even apoptosis. On the basis of their biological properties the compounds according to the invention, their isomers and the physiologically acceptable salts thereof are suitable for treating diseases characterised by excessive or abnormal cell proliferation.

### Example Aurora-B Kinase Assay

A radioactive enzyme inhibition assay was developed using E.coli-expressed recombinant *Xenopus laevis* Aurora B wild-type protein equipped at the N-terminal position with a GST tag (amino acids 60-361) in a complex with *Xenopus laevis* INCENP (amino acids 790-847), which is obtained from bacteria and purified. In equivalent manner a *Xenopus laevis* Aurora B mutant (G96V) in a complex with *Xenopus laevis* INCENP⁷⁹⁰⁻⁸⁴⁷ may also be used.

### Expression and purification

The coding sequence for Aurora-B⁶⁰⁻³⁶¹ from *Xenopus laevis* is cloned into a modified version of pGEX-6T (Amersham Biotech) via BamHI and SalI cutting sites. The vector contains two cloning cassettes which are separated by a ribosomal binding site, allowing bi-cistronic expression. In this configuration *Xenopus laevis* Aurora B is expressed by the first cassette, and the *Xenopus laevis* INCENP⁷⁹⁰⁻⁸⁴⁷ is expressed by the second cassette. The resulting vector is pAUB-IN⁸⁴⁷.

First of all the E.coli strain BL21 (DE3) is co-transformed with pUBS520 helper plasmid and pAUB-IN⁸⁴⁷, after which protein expression is induced using 0.3 mM IPTG at an OD₆₀₀ of 0.45-0.7. The expression is then continued for approx. 12-16 hours at 23-25°C with agitation.

The bacteria are then removed by centrifuging and the pellet is lysed in lysis buffer (50 mM Tris/Cl pH 7.6, 300 mM NaCl, 1 mM DTT, 1 mM EDTA, 5 % glycerol, Roche Complete Protease Inhibitor tablets) using ultrasound, using 20-30 mL lysis buffer per litre of E.coli culture. The lysed material is freed from debris by centrifugation (12000 rpm, 45-60 min, JA20 rotor). The supernatant is incubated with 300 µL of equilibrated GST Sepharose Fast Flow (Amersham Biosciences) per litre of E.coli culture for 4-5 h at 4°C. Then the column material is washed with 30 volumes of lysis buffer and then equilibrated with 30 volumes of cleavage buffer (50 mM Tris/Cl pH 7.6, 150 mM NaCl, 1 mM DTT, 1 mM EDTA). To cleave the GST tag from Aurora B, 10 units of Prescission Protease (Amersham Biosciences) are used per milligram of substrate and the mixture is incubated for 16 h at 4°C. The supernatant which contains the cleavage product is loaded onto a 6 mL Resource Q column (Amersham Biosciences) equilibrated with ion exchange buffer (50 mM Tris/Cl pH 7.6, 150 mM NaCl, 1 mM DTT, 1 mM EDTA). The Aurora B/INCENP complex is caught as it flows through, then concentrated and loaded onto a Superdex 200 size exclusion chromatography (SEC) column equilibrated with SEC buffer (10 mM Tris/Cl pH 7.6, 150 mM NaCl, 1 mM DTT, 1 mM EDTA). Fractions which contain the AuroraB /INCENP complex are collected and concentrated using Vivaspin concentrators (molecular weight exclusion 3000-5000 Da) to a final concentration of 12 mg/mL. Aliquots (e.g. 240 ng/µL) for kinase assays are transferred from this stock solution into freezing buffer (50 mM Tris/Cl pH 8.0, 150 mM NaCl, 0.1 mM EDTA, 0.03% Brij-35, 10% glycerol, 1 mM DTT) and stored at -80°C.

### Kinase Assay

Test substances are placed in a polypropylene dish (96 wells, Greiner #655 201), in order to cover a concentration frame of 10 µM - 0.0001 µM. The final concentration of DMSO in the assay is 5%. 30 µL of protein mix (50 mM tris/Cl pH 7.5, 25 mM MgCl₂, 25 mM NaCl, 167 µM ATP, 10 ng Xenopus laevis Aurora B/INCENP complex in freezing buffer) are pipetted into the 10 µl of test substance provided in 25% DMSO and this is incubated for 15 min at RT. Then 10 µL of peptide mix (100 mM tris/Cl pH 7.5, 50 mM MgCl₂, 50 mM NaCl, 5 µM NaF, 5 µM DTT, 1 µCi gamma-P33-ATP [Amersham], 50 µM substrate peptide [biotin-EPLERRLSLVPDS or multimers thereof, or biotin-EPLERRLSLVPKM or multimers thereof, or biotin-LRRWSLGLRRWSLGLRRWSLGLRRWSLG]) are added. The reaction is incubated for 75 min (ambient temperature) and stopped by the addition of 180 µL of 6.4% trichloroacetic acid and incubated for 20 min on ice. A multiscreen filtration plate (Millipore, MAIP NOB10) is equilibrated first of all with 100 µL 70% ethanol and then with 180 µL trichloroacetic acid and the liquids are eliminated using a suitable suction apparatus. Then the stopped kinase reaction is applied. After 5 washing steps with 180 µL 1% trichloroacetic acid in each case the lower half of the dish is dried (10-20 min at 55°C) and 25 µL scintillation cocktail (Microscint, Packard # 6013611) is added. Incorporated gamma-phosphate is quantified using a Wallac 1450 Microbeta Liquid Scintillation Counter. Samples without test substance or without substrate peptide are used as controls. IC₅₀ values are obtained using Graph Pad Prism software.

The anti-proliferative activity of the compounds according to the invention is determined in the proliferation test on cultivated human tumour cells and/or in a cell cycle analysis, for example on NCI-H460 tumour cells. In both test methods compounds 1 - 41 exhibit good to very good activity, i.e. for example an EC50 value in the NCI-H460 proliferation test of less than 1 µmol/L, generally less than 0.1 µmol/L.

### Measurement of the inhibition of proliferation on cultivated human tumour cells

To measure proliferation on cultivated human tumour cells, cells of lung tumour cell line NCI-H460 (obtained from American Type Culture Collection (ATCC)) are cultivated in RPMI 1640 medium (Gibco) and 10% foetal calf serum (Gibco) and harvested in the log growth phase. Then the NCI-H460 cells are placed in 96-well flat-bottomed plates (Falcon) at a density of 1000 cells per well in RPMI 1640 medium and incubated overnight in an incubator (at 37°C and 5 % CO₂). The active substances are added to the cells in various concentrations (dissolved in DMSO; DMSO final concentration: 0.1%). After 72 hours incubation 20 µl AlamarBlue reagent (AccuMed International) is added to each well, and the cells are incubated for a further 5-7 hours. After incubation the colour change of the AlamarBlue reagent is determined in a Wallac Microbeta fluorescence spectrophotometer. EC₅₀ values are calculated using Standard Levenburg Marquard algorithms (GraphPadPrizm).

Cell cycle analyses are carried out for example using FACS analyses (Fluorescence Activated Cell Sorter) or by Cellomics Array Scan (CellCycle Analysis) .

### FACS Analysis

Propidium iodide (PI) binds stoichiometrically to double-stranded DNA, and is thus suitable for determining the proportion of cells in the G1, S, and G2/M phase of the cell cycle on the basis of the cellular DNA content. Cells in the G0 and G1 phase have a diploid DNA content (2N), whereas cells in the G2 or mitosis phase have a 4N DNA content.

For PI staining, for example, 1.75x10⁶ NCI-H460 cells are seeded onto a 75 cm² cell culture flask, and after 24 h either 0.1 % DMSO is added as control or the substance is added in various concentrations (in 0.1% DMSO). The cells are incubated for 42 h with the substance or with DMSO. Then the cells are detached with trypsin and centrifuged. The cell pellet is washed with buffered saline solution (PBS) and the cells are then fixed with 80% at -20°C for at least 2 h. After another washing step with PBS the cells are permeabilised with Triton X-100 (Sigma; 0.25% in PBS) on ice for 5 min, and then incubated with a solution of propidium iodide (Sigma; 10µg/ml) and RNAse (Serva; 1mg/mLl) in the ratio 9:1 1 for at least 20 min in the dark.

The DNA measurement is carried out in a Becton Dickinson FACS Analyzer, with an argon laser (500 mW, emission 488 nm); data are obtained and evaluated using the DNA Cell Quest Programme (BD).

### Cellomics Array Scan

NCI-H460 cells are seeded into 96-well flat-bottomed dishes (Falcon) in RPMI 1640 medium (Gibco) with 10% foetal calf serum (Gibco) in a density of 2000 cells per well and incubated overnight in an incubator (at 37°C and 5 % CO₂). The active substances are added to the cells in various concentrations (dissolved in DMSO; DMSO final concentration: 0.1%). After 42 h incubation the medium is suction filtered, the cells are fixed for 10 min with 4% formaldehyde solution and Triton X-100 (1:200 in PBS) at ambient temperature and simultaneously permeabilised, and then washed twice with a 0.3% BSA solution (Calbiochem). Then the DNA is stained by the addition of 50 µL/well of 4',6-diamidino-2-phenylindole (DAPI; Molecular Probes) in a final concentration of 300 nM for 1 h at ambient temperature, in the dark. The preparations are then carefully washed twice with PBS, the plates are stuck down with black adhesive film and analysed in the Cellomics ArrayScan using the CellCycle BioApplication programme and visualised and evaluated using Spotfire.

The substances of the present invention are Aurora kinase inhibitors. On the basis of their biological properties the compounds according to the invention, their isomers and the physiologically acceptable salts thereof are suitable for treating diseases characterised by excessive or abnormal cell proliferation.

Such diseases include for example: viral infections (e.g. HIV and Kaposi's sarcoma); inflammatory and autoimmune diseases (e.g. colitis, arthritis, Alzheimer's disease, glomerulonephritis and wound healing); bacterial, fungal and/or parasitic infections; leukaemias, lymphomas and solid tumours (e.g. carcinomas and sarcomas), skin diseases (e.g. psoriasis); diseases based on hyperplasia which are characterised by an increase in the number of cells (e.g. fibroblasts, hepatocytes, bones and bone marrow cells, cartilage or smooth muscle cells or epithelial cells (e.g. endometrial hyperplasia)); bone diseases and cardiovascular diseases (e.g. restenosis and hypertrophy).

For example, the following cancers may be treated with compounds according to the invention, without being restricted thereto: brain tumours such as for example acoustic neurinoma, astrocytomas such as pilocytic astrocytomas, fibrillary astrocytoma, protoplasmic astrocytoma, gemistocytary astrocytoma, anaplastic astrocytoma and glioblastoma, brain lymphomas, brain metastases, hypophyseal tumour such as prolactinoma, HGH (human growth hormone) producing tumour and ACTH producing tumour (adrenocorticotropic hormone), craniopharyngiomas, medulloblastomas, meningeomas and oligodendrogliomas; nerve tumours (neoplasms) such as for example tumours of the vegetative nervous system such as neuroblastoma sympathicum, ganglioneuroma, paraganglioma (pheochromocytoma, chromaffinoma) and glomus-caroticum tumour, tumours on the peripheral nervous system such as amputation neuroma, neurofibroma, neurinoma (neurilemmoma, Schwannoma) and malignant Schwannoma, as well as tumours of the central nervous system such as brain and bone marrow tumours; intestinal cancer such as for example carcinoma of the rectum, colon, anus, small intestine and duodenum; eyelid tumours such as basalioma or basal cell carcinoma; pancreatic cancer or carcinoma of the pancreas; bladder cancer or carcinoma of the bladder; lung cancer (bronchial carcinoma) such as for example small-cell bronchial carcinomas (oat cell carcinomas) and non-small cell bronchial carcinomas such as plate epithelial carcinomas, adenocarcinomas and large-cell bronchial carcinomas; breast cancer such as for example mammary carcinoma such as infiltrating ductal carcinoma, colloid carcinoma, lobular invasive carcinoma, tubular carcinoma, adenocystic carcinoma and papillary carcinoma; non-Hodgkin's lymphomas (NHL) such as for example Burkitt's lymphoma, low-malignancy non-Hodgkin's lymphomas (NHL) and mucosis fungoides; uterine cancer or endometrial carcinoma or corpus carcinoma; CUP syndrome (Cancer of Unknown Primary); ovarian cancer or ovarian carcinoma such as mucinous, endometrial or serous cancer; gall bladder cancer; bile duct cancer such as for example Klatskin tumour; testicular cancer such as for example seminomas and non-seminomas; lymphoma (lymphosarcoma) such as for example malignant lymphoma, Hodgkin's disease, non-Hodgkin's lymphomas (NHL) such as chronic lymphatic leukaemia, leukaemic reticuloendotheliosis, immunocytoma, plasmocytoma (multiple myeloma), immunoblastoma, Burkitt's lymphoma, T-zone mycosis fungoides, large-cell anaplastic lymphoblastoma and lymphoblastoma; laryngeal cancer such as for example tumours of the vocal cords, supraglottal, glottal and subglottal laryngeal tumours; bone cancer such as for example osteochondroma, chondroma, chondroblastoma, chondromyxoid fibroma, osteoma, osteoid osteoma, osteoblastoma, eosinophilic granuloma, giant cell tumour, chondrosarcoma, osteosarcoma, Ewing's sarcoma, reticulo-sarcoma, plasmocytoma, giant cell tumour, fibrous dysplasia, juvenile bone cysts and aneurysmatic bone cysts; head and neck tumours such as for example tumours of the lips, tongue, floor of the mouth, oral cavity, gums, palate, salivary glands, throat, nasal cavity, paranasal sinuses, larynx and middle ear; liver cancer such as for example liver cell carcinoma or hepatocellular carcinoma (HCC); leukaemias, such as for example acute leukaemias such as acute lymphatic/lymphoblastic leukaemia (ALL), acute myeloid leukaemia (AML); chronic leukaemias such as chronic lymphatic leukaemia (CLL), chronic myeloid leukaemia (CML); stomach cancer or gastric carcinoma such as for example papillary, tubular and mucinous adenocarcinoma, signet ring cell carcinoma, adenosquamous carcinoma, small-cell carcinoma and undifferentiated carcinoma; melanomas such as for example superficially spreading, nodular, lentigo-maligna and acral-lentiginous melanoma; renal cancer such as for example kidney cell carcinoma or hypernephroma or Grawitz's tumour; oesophageal cancer or carcinoma of the oesophagus; penile cancer; prostate cancer; throat cancer or carcinomas of the pharynx such as for example nasopharynx carcinomas, oropharynx carcinomas and hypopharynx carcinomas; retinoblastoma; vaginal cancer or vaginal carcinoma; plate epithelial carcinomas, adenocarcinomas, in situ carcinomas, malignant melanomas and sarcomas; thyroid carcinomas such as for example papillary, follicular and medullary thyroid carcinoma, as well as anaplastic carcinomas; spinalioma, epidormoid carcinoma and plate epithelial carcinoma of the skin; thymomas, cancer of the urethra and cancer of the vulva.

The new compounds may be used for the prevention, short-term or long-term treatment of the above-mentioned diseases, optionally also in combination with radiotherapy or other "state-of-the-art" compounds, such as e.g. cytostatic or cytotoxic substances, cell proliferation inhibitors, anti-angiogenic substances, steroids or antibodies.

The compounds may be used on their own or in combination with other active substances according to the invention, optionally also in combination with other pharmacologically active substances.

Chemotherapeutic agents which may be administered in combination with the compounds according to the invention, include, without being restricted thereto, hormones, hormone analogues and antihormones (e.g. tamoxifen, toremifene, raloxifene, fulvestrant, megestrol acetate, flutamide, nilutamide, bicalutamide, aminoglutethimide, cyproterone acetate, finasteride, buserelin acetate, fludrocortinsone, fluoxymesterone, medroxyprogesterone, octreotide), aromatase inhibitors (e.g. anastrozole, letrozole, liarozole, vorozole, exemestane, atamestane), LHRH agonists and antagonists (e.g. goserelin acetate, luprolide), inhibitors of growth factors (growth factors such as for example "platelet derived growth factor" and "hepatocyte growth factor", inhibitors are for example "growth factor" antibodies, "growth factor receptor" antibodies and tyrosinekinase inhibitors, such as for example gefitinib, imatinib, lapatinib cetuximab (Erbitux ®) and trastuzumab); antimetabolites (e.g. antifolates such as methotrexate, raltitrexed, pyrimidine analogues such as 5-fluorouracil, capecitabin and gemcitabin, purine and adenosine analogues such as mercaptopurine, thioguanine, cladribine and pentostatin, cytarabine, fludarabine); antitumour antibiotics (e.g. anthracyclins such as doxorubicin, daunorubicin, epirubicin and idarubicin, mitomycin-C, bleomycin, dactinomycin, plicamycin, streptozocin); platinum derivatives (e.g. cisplatin, oxaliplatin, carboplatin); alkylation agents (e.g. estramustin, meclorethamine, melphalan, chlorambucil, busulphan, dacarbazin, cyclophosphamide, ifosfamide, temozolomide, nitrosoureas such as for example carmustin and lomustin, thiotepa); antimitotic agents (e.g. vinca alkaloids such as for example vinblastine, vindesin, vinorelbin and vincristine; and taxanes such as paclitaxel, docetaxel); topoisomerase inhibitors (e.g. epipodophyllotoxins such as for example etoposide and etopophos, teniposide, amsacrin, topotecan, irinotecan, mitoxantron) and various chemotherapeutic agents such as amifostin, anagrelid, clodronat, filgrastin, interferon alpha, leucovorin, rituximab, procarbazine, levamisole, mesna, mitotane, pamidronate and porfimer.

Suitable preparations include for example tablets, capsules, suppositories, solutions, - particularly solutions for injection (s.c., i.v., i.m.) and infusion - elixirs, emulsions or dispersible powders. The content of the pharmaceutically active compound(s) should be in the range from 0.1 to 90 wt.-%, preferably 0.5 to 50 wt.-% of the composition as a whole, i.e. in amounts which are sufficient to achieve the dosage range specified below. The doses specified may, if necessary, be given several times a day.

Suitable tablets may be obtained, for example, by mixing the active substance(s) with known excipients, for example inert diluents such as calcium carbonate, calcium phosphate or lactose, disintegrants such as corn starch or alginic acid, binders such as starch or gelatine, lubricants such as magnesium stearate or talc and/or agents for delaying release, such as carboxymethyl cellulose, cellulose acetate phthalate, or polyvinyl acetate. The tablets may also comprise several layers.

Coated tablets may be prepared accordingly by coating cores produced analogously to the tablets with substances normally used for tablet coatings, for example collidone or shellac, gum arabic, talc, titanium dioxide or sugar. To achieve delayed release or prevent incompatibilities the core may also consist of a number of layers. Similarly the tablet coating may consist of a number of layers to achieve delayed release, possibly using the excipients mentioned above for the tablets.

Syrups or elixirs containing the active substances or combinations thereof according to the invention may additionally contain a sweetener such as saccharine, cyclamate, glycerol or sugar and a flavour enhancer, e.g. a flavouring such as vanillin or orange extract. They may also contain suspension adjuvants or thickeners such as sodium carboxymethyl cellulose, wetting agents such as, for example, condensation products of fatty alcohols with ethylene oxide, or preservatives such as p-hydroxybenzoates.

Solutions for injection and infusion are prepared in the usual way, e.g. with the addition of isotonic agents, preservatives such as p-hydroxybenzoates, or stabilisers such as alkali metal salts of ethylenediamine tetraacetic acid, optionally using emulsifiers and/or dispersants, whilst if water is used as the diluent, for example, organic solvents may optionally be used as solvating agents or dissolving aids, and transferred into injection vials or ampoules or infusion bottles.

Capsules containing one or more active substances or combinations of active substances may for example be prepared by mixing the active substances with inert carriers such as lactose or sorbitol and packing them into gelatine capsules.

Suitable suppositories may be made for example by mixing with carriers provided for this purpose, such as neutral fats or polyethyleneglycol or the derivatives thereof

Excipients which may be used include, for example, water, pharmaceutically acceptable organic solvents such as paraffins (e.g. petroleum fractions), vegetable oils (e.g. groundnut or sesame oil), mono- or polyfunctional alcohols (e.g. EtOH or glycerol), carriers such as e.g. natural mineral powders (e.g. kaolins, clays, talc, chalk), synthetic mineral powders (e.g. highly dispersed silicic acid and silicates), sugars (e.g. cane sugar, lactose and glucose) emulsifiers (e.g. lignin, spent sulphite liquors, methylcellulose, starch and polyvinylpyrrolidone) and lubricants (e.g. magnesium stearate, talc, stearic acid and sodium lauryl sulphate).

The preparations are administered by the usual methods, preferably by oral or transdermal route, most preferably by oral route. For oral administration the tablets may, of course contain, apart from the abovementioned carriers, additives such as sodium citrate, calcium carbonate and dicalcium phosphate together with various additives such as starch, preferably potato starch, gelatine and the like. Moreover, lubricants such as magnesium stearate, sodium lauryl sulphate and talc may be used at the same time for the tabletting process. In the case of aqueous suspensions the active substances may be combined with various flavour enhancers or colourings in addition to the excipients mentioned above.

For parenteral use, solutions of the active substances with suitable liquid carriers may be used.

The dosage for intravenous use is from 1 - 1000 mg per hour, preferably between 5 and 500 mg per hour.

However, it may sometimes be necessary to depart from the amounts specified, depending on the body weight, the route of administration, the individual response to the drug, the nature of its formulation and the time or interval over which the drug is administered. Thus, in some cases it may be sufficient to use less than the minimum dose given above, whereas in other cases the upper limit may have to be exceeded. When administering large amounts it may be advisable to divide them up into a number of smaller doses spread over the day.

### Examples of pharmaceutical formulations

A)

| Tablets | per tablet |
|---|---|
| active substance according to formula (1) | 100 mg |
| lactose | 140 mg |
| corn starch | 240 mg |
| polyvinylpyrrolidone | 15 mg |
| magnesium stearate | 5 mg |
| | 500 mg |

The finely ground active substance, lactose and some of the corn starch are mixed together. The mixture is screened, then moistened with a solution of polyvinylpyrrolidone in water, kneaded, wet-granulated and dried. The granules, the remaining corn starch and the magnesium stearate are screened and mixed together. The mixture is compressed to produce tablets of suitable shape and size.
B)

| Tablets | per tablet |
|---|---|
| active substance according to formula (1) | 80 mg |
| lactose | 55 mg |
| corn starch | 190 mg |
| microcrystalline cellulose | 35 mg |
| polyvinylpyrrolidone | 15 mg |
| sodium-carboxymethyl starch | 23 mg |
| magnesium stearate | 2 mg |
| | 400 mg |

The finely ground active substance, some of the corn starch, lactose, microcrystalline cellulose and polyvinylpyrrolidone are mixed together, the mixture is screened and worked with the remaining corn starch and water to form a granulate which is dried and screened. The sodiumcarboxymethyl starch and the magnesium stearate are added and mixed in and the mixture is compressed to form tablets of a suitable size.
C) Ampoule solution

| | |
|---|---|
| active substance | 50 mg |
| sodium chloride | 50 mg |
| water for inj. | 5 ml |

The active substance is dissolved in water at its own pH or optionally at pH 5.5 to 6.5 and sodium chloride is added to make it isotonic. The solution obtained is filtered free from pyrogens and the filtrate is transferred under aseptic conditions into ampoules which are then sterilised and sealed by fusion. The ampoules contain 5 mg, 25 mg and 50 mg of active substance.

## Claims

1. Compounds selected from the group consisting of:

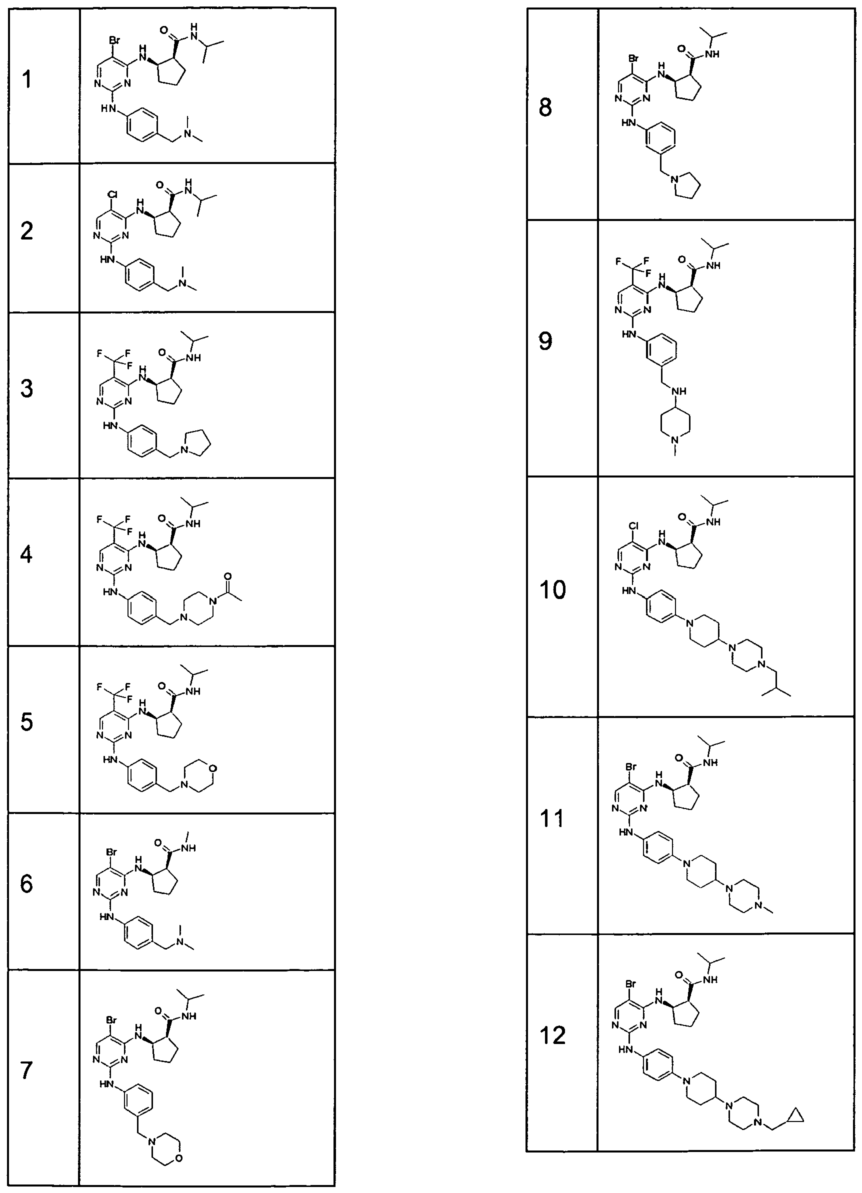

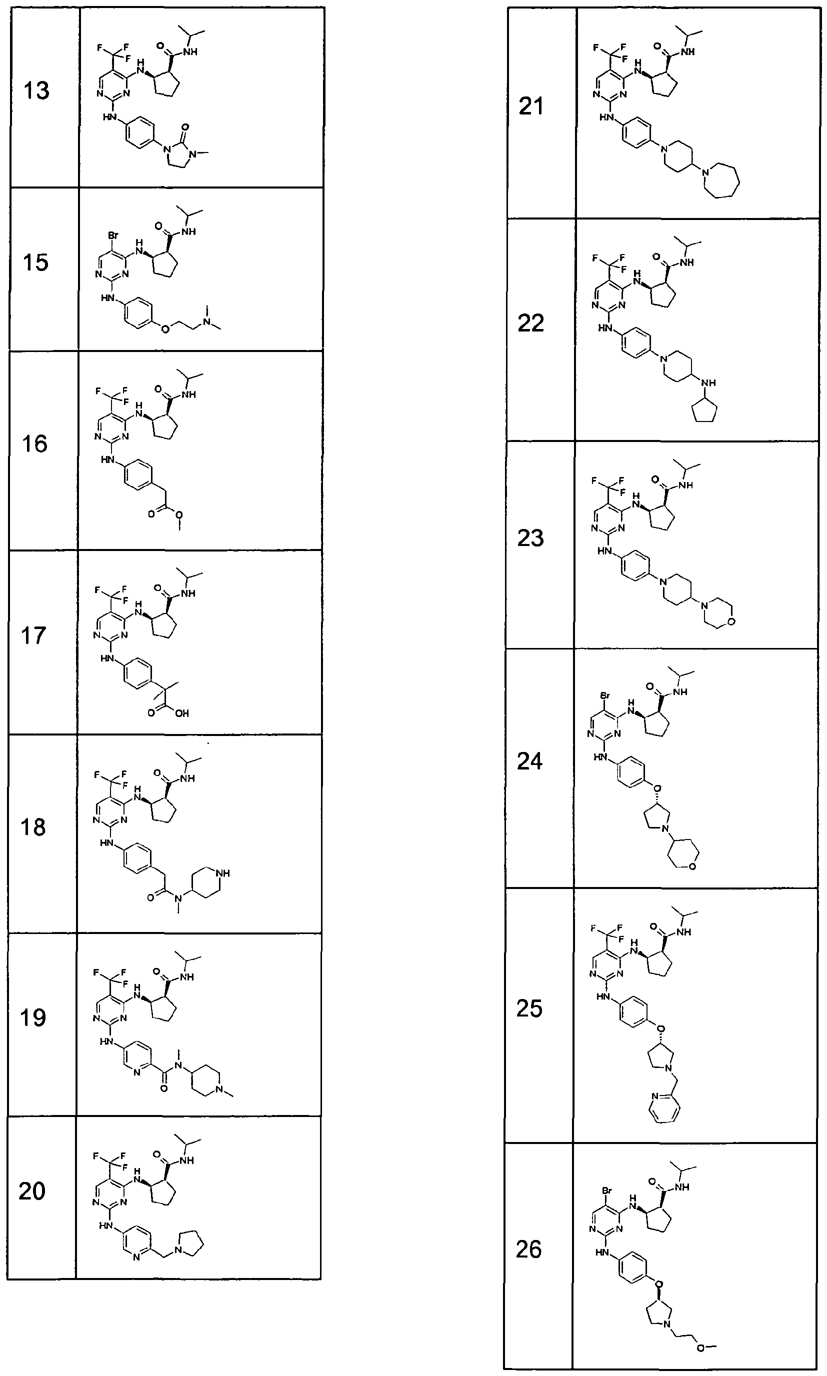

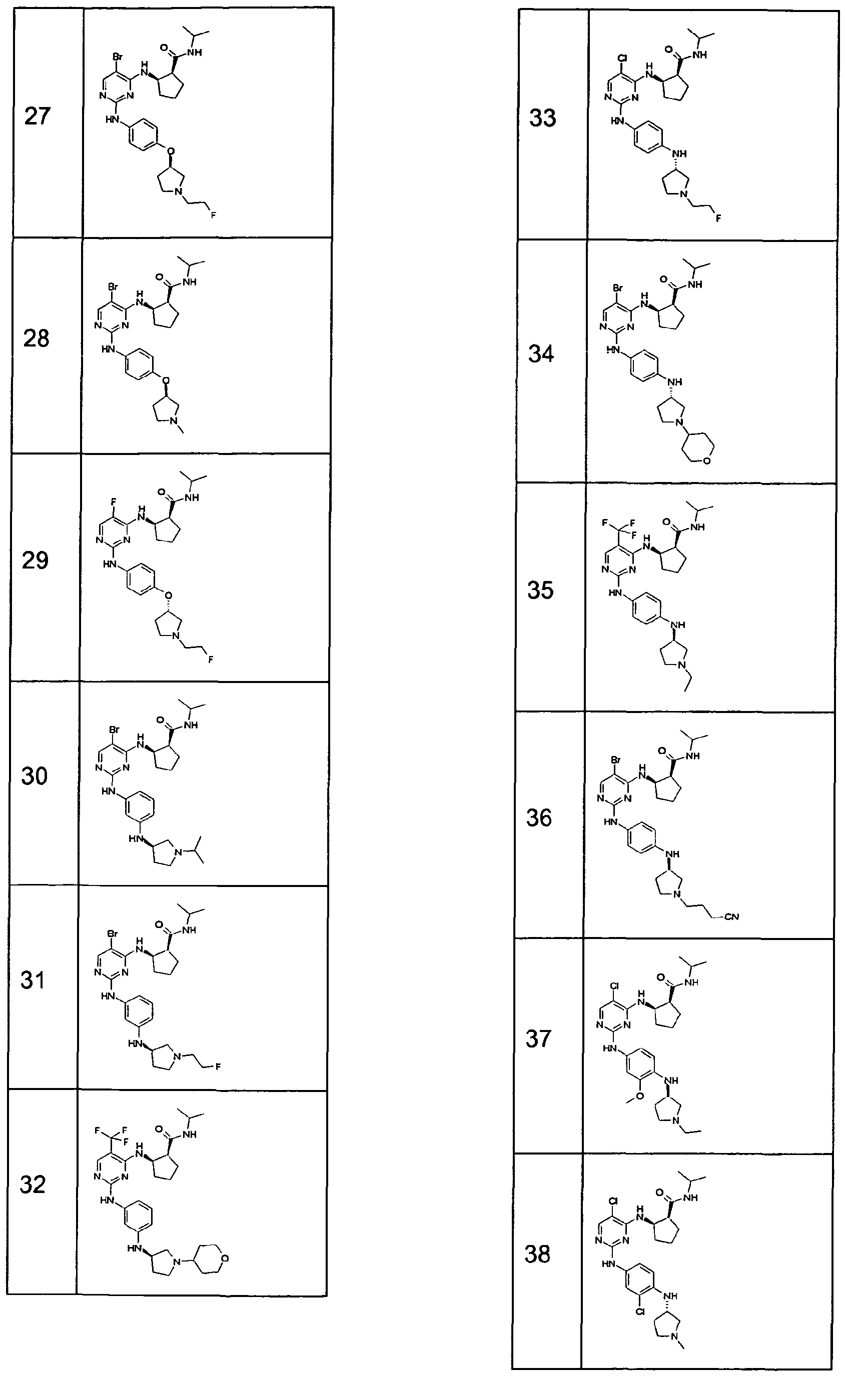

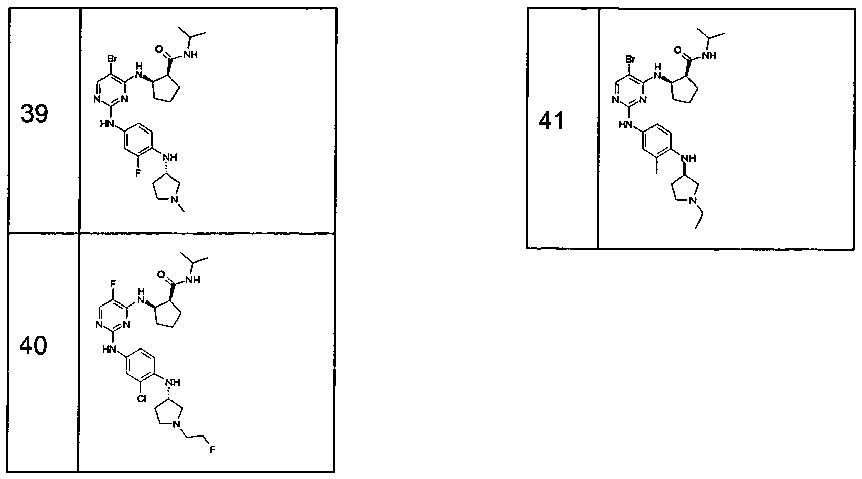

2. Compounds, or the pharmaceutically effective salts thereof, according to claim 1 for use as pharmaceutical compositions.

3. Compounds, or the pharmaceutically effective salts thereof, according to claim 1 for preparing a pharmaceutical composition with an antiproliferative activity.

4. Pharmaceutical preparations, containing as active substance one or more compounds according to claim 1 or the pharmaceutically effective salts thereof, optionally in combination with conventional excipients and/or carriers.

5. Use of compounds according to claim 1 for preparing a pharmaceutical composition for the treatment and/or prevention of cancer, infections, inflammations and autoimmune diseases.

6. Pharmaceutical preparation comprising a compound according to claim 1 and at least one other cytostatic or cytotoxic active substance different from a compound of claim 1, optionally in the form of the tautomers, the racemates, the enantiomers, the diastereomers and the mixtures thereof, and optionally the pharmaceutically effective salts thereof

## Patentansprüche

1. Verbindungen ausgewählt aus der Gruppe bestehend aus:

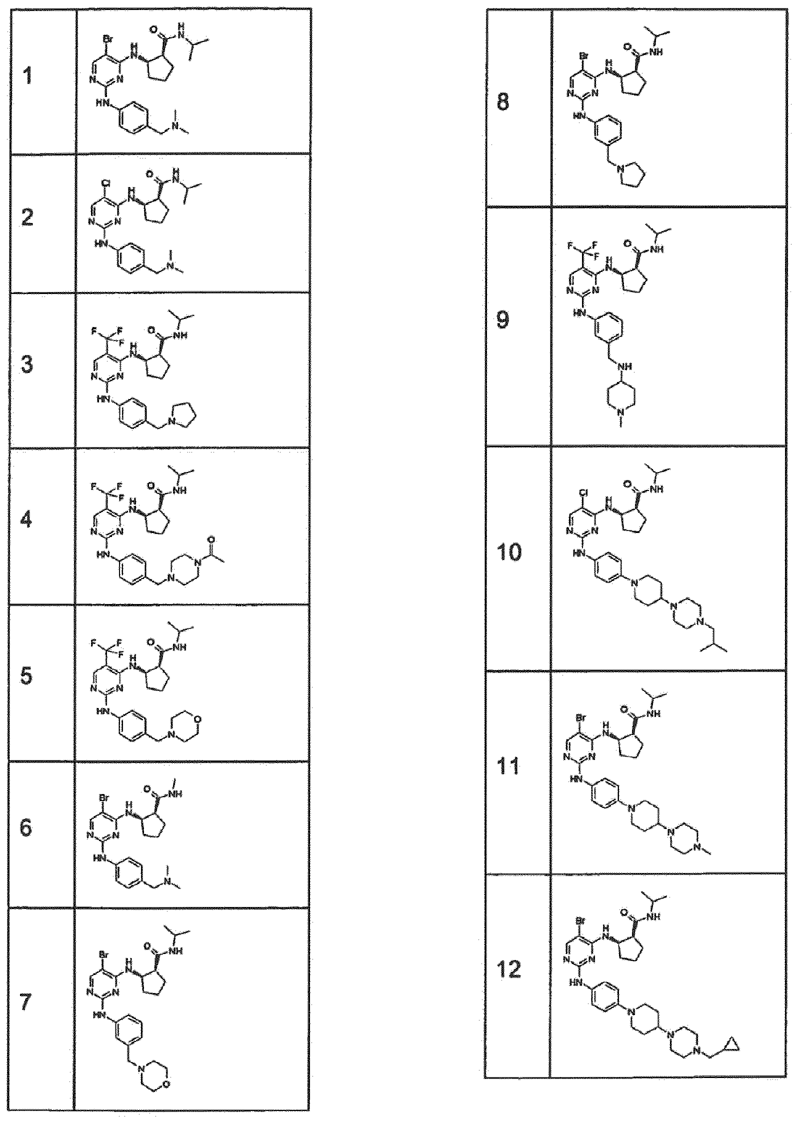

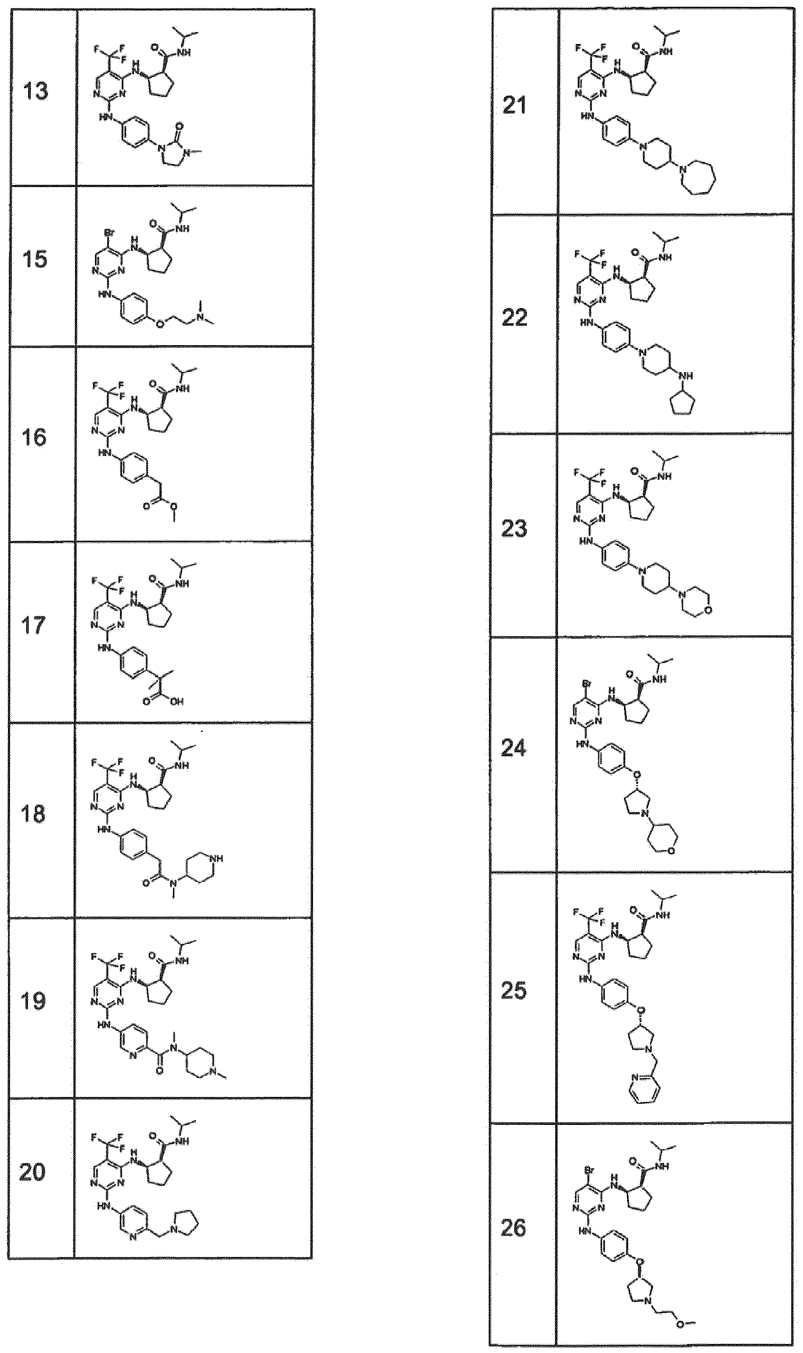

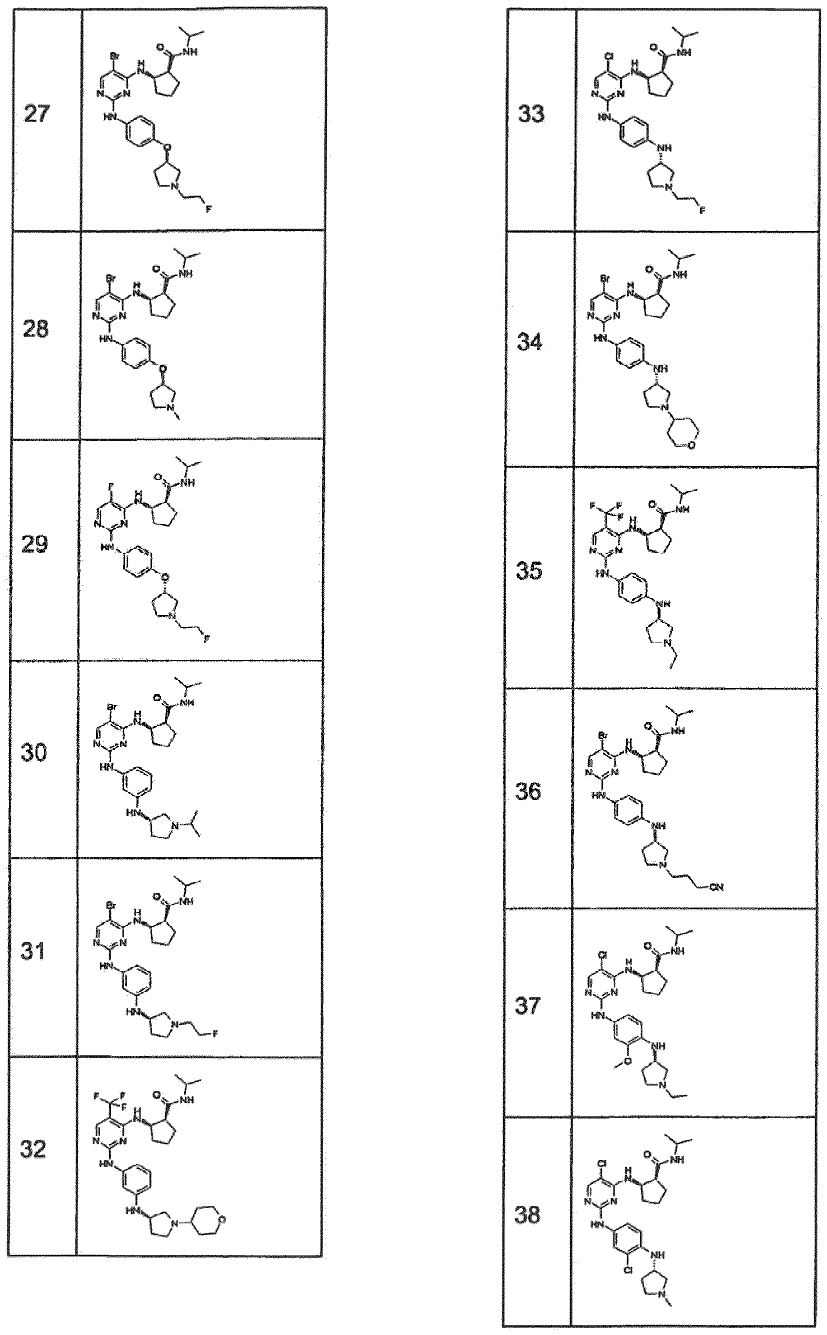

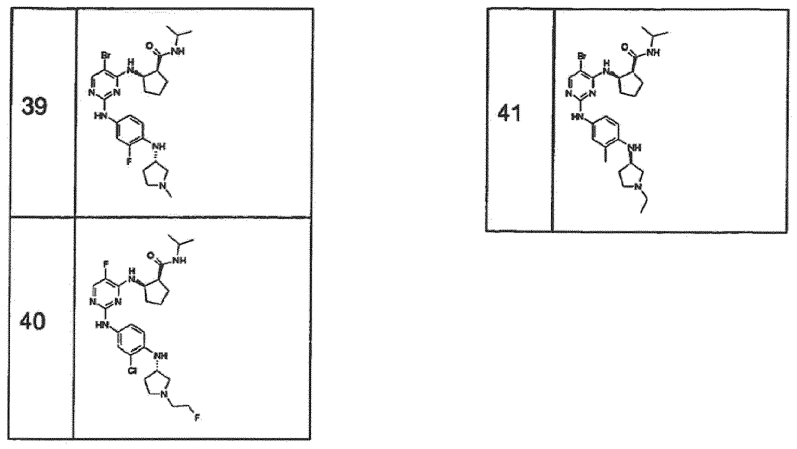

2. Verbindungen, oder die pharmazeutisch wirksamen Salze davon, nach Anspruch 1 zur Verwendung als pharmazeutische Zusammensetzungen.

3. Verbindungen, oder die pharmazeutisch wirksamen Salze davon, nach Anspruch 1 zur Herstellung einer pharmazeutischen Zusammensetzung mit antiproliferativer Aktivität.

4. Pharmazeutische Zubereitungen, enthaltend als Wirkstoff eine oder mehrere Verbindungen nach Anspruch 1 oder die pharmazeutisch wirksamen Salze davon, gegebenenfalls in Kombination mit herkömmlichen Exzipienten und/oder Trägern.

5. Verwendung von Verbindungen nach Anspruch 1 zur Herstellung einer pharmazeutischen Zusammensetzung zur Behandlung und/oder Vorbeugung von Krebs, Infektionen, Entzündungen und Autoimmunerkrankungen.

6. Pharmazeutische Zubereitung, umfassend eine Verbindung nach Anspruch 1 und mindestens einen anderen zytostatischen oder zytotoxischen Wirkstoff, der sich von einer Verbindung nach Anspruch 1 unterscheidet, gegebenenfalls in Form der Tautomeren, der Racemate, der Enantiomere, der Diastereomere und der Mischungen davon, und gegebenenfalls der pharmazeutisch wirksamen Salzen davon.

## Revendications

1. Composés choisis dans le groupe consistant en

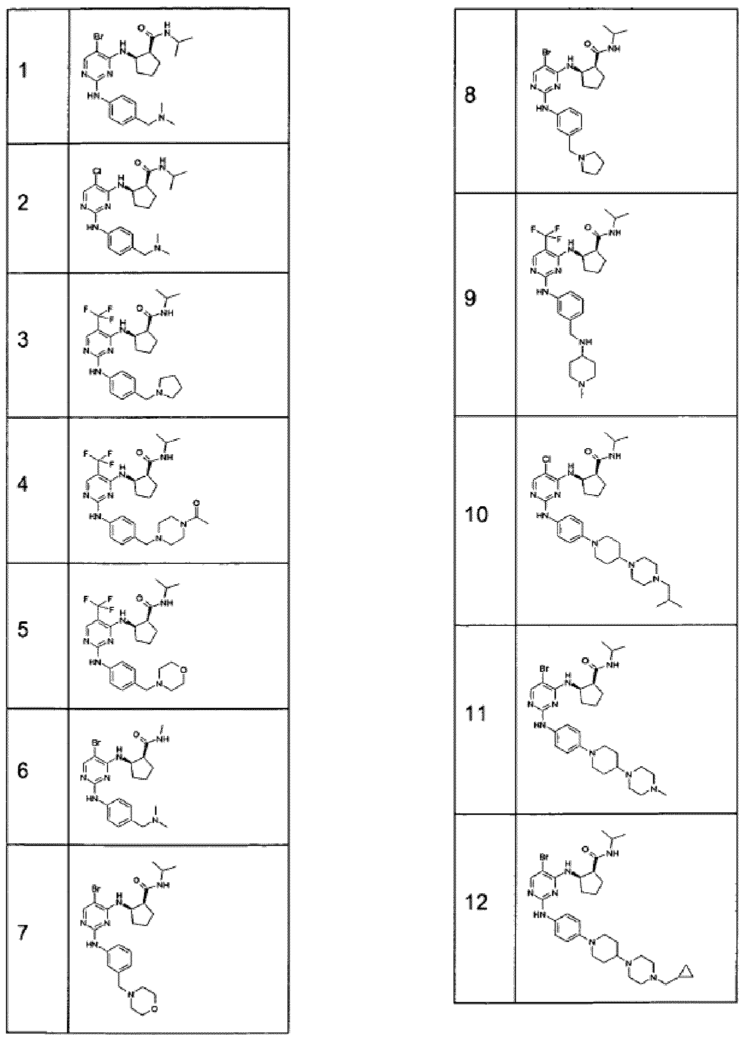

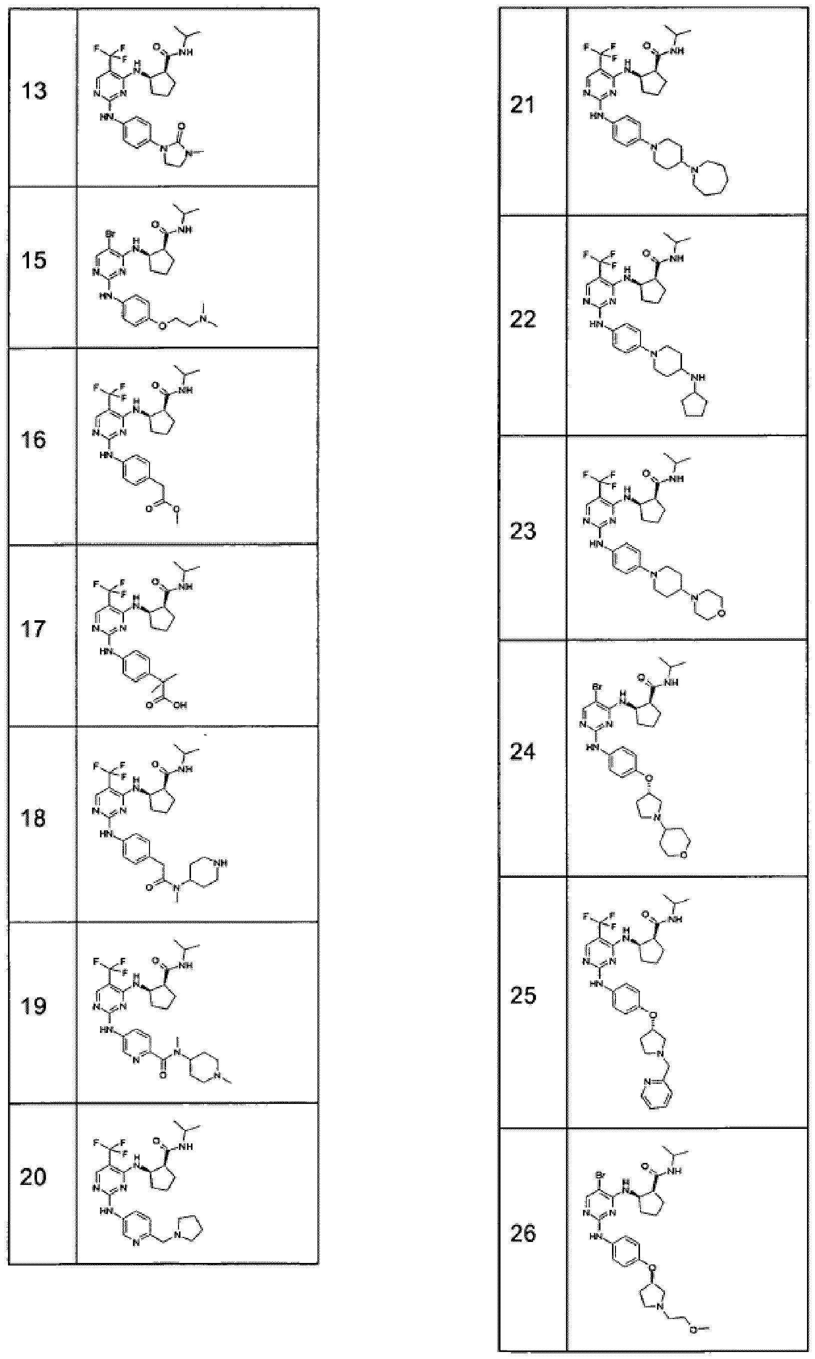

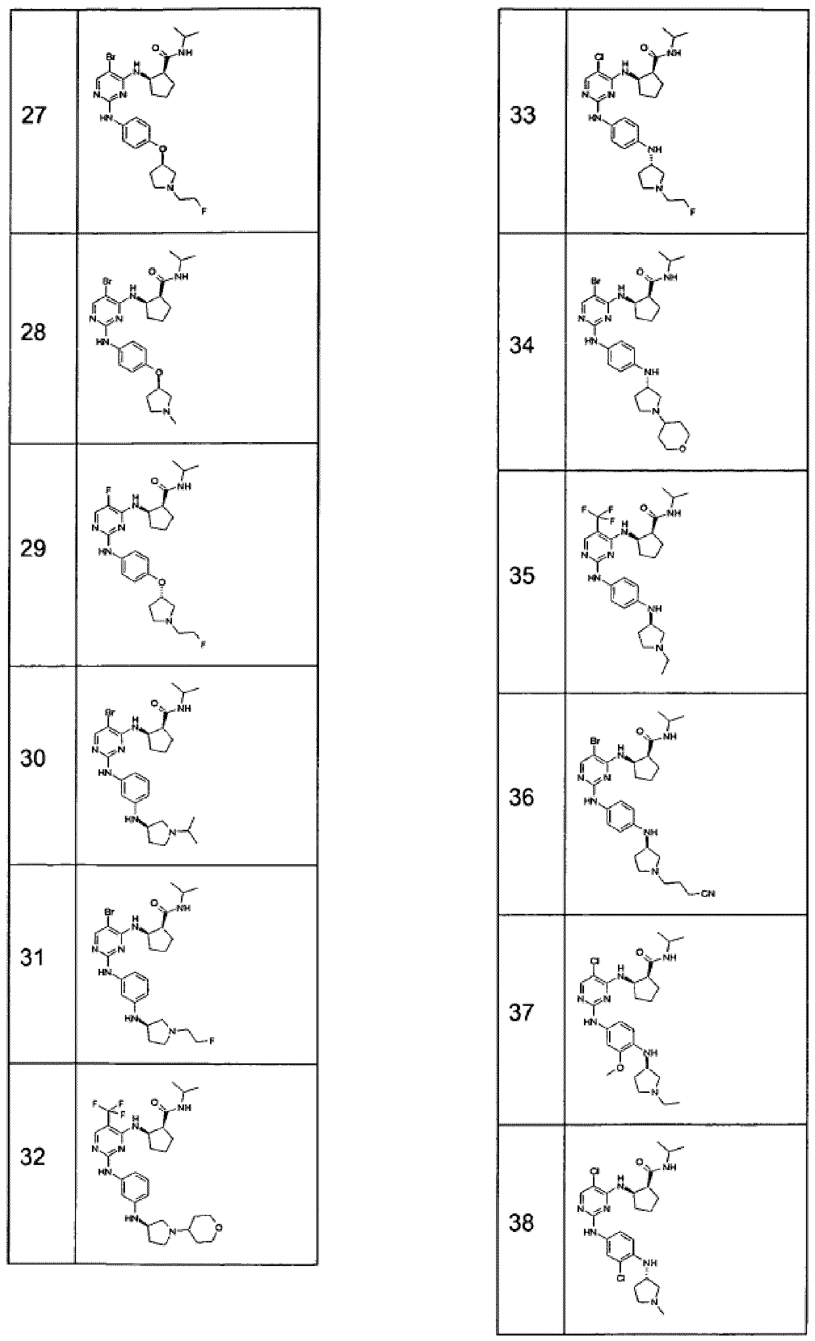

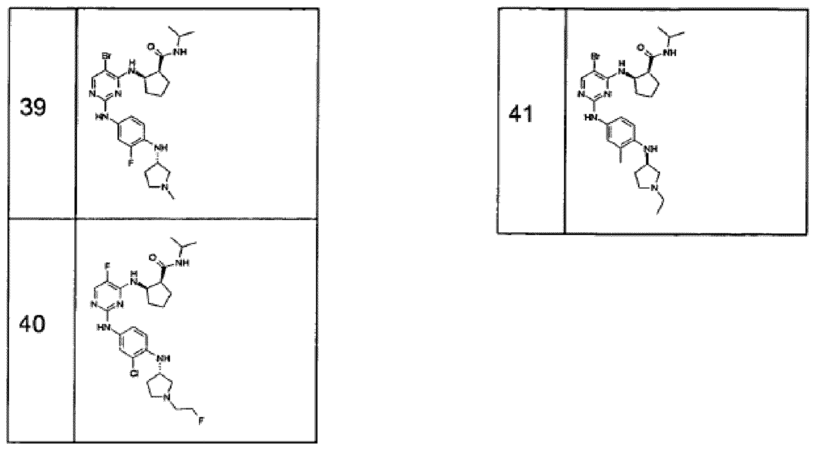

2. Composés, ou sels pharmaceutiquement efficaces de ceux-ci, selon la revendication 1 pour une utilisation en tant que compositions pharmaceutiques.

3. Composés, ou sels pharmaceutiquement efficaces de ceux-ci, selon la revendication 1 pour la préparation d'une composition pharmaceutique avec une activité antiproliférative.

4. Préparations pharmaceutiques, contenant en tant que substance active un ou plusieurs composés selon la revendication 1 ou les sels pharmaceutiquement efficaces de ceux-ci, éventuellement en combinaison avec des excipients et/ou des supports traditionnels.

5. Utilisation de composés selon la revendication 1 pour la préparation d'une composition pharmaceutique destinée au traitement et/ou à la prévention d'un cancer, d'infections, d'inflammations et de maladies auto-immunes.

6. Préparation pharmaceutique comprenant un composé selon la revendication 1 et au moins une autre substance active cytostatique ou cytotoxique différente d'un composé selon la revendication 1, éventuellement sous la forme des tautomères, des racémates, des énantiomères, des diastéréomères et des mélanges de ceux-ci, et éventuellement les sels pharmaceutiquement efficaces de ceux-ci.
